(19) **Europäisches Patentamt**
**European Patent Office**
**Office européen des brevets**

(11) **EP 2 502 617 B1**

(12) **EUROPEAN PATENT SPECIFICATION**

(45) Date of publication and mention
of the grant of the patent:
**20.03.2019 Bulletin 2019/12**

(21) Application number: **10831526.8**

(22) Date of filing: **15.11.2010**

(51) Int Cl.:
*A61K 9/113* (2006.01)        *A61K 47/44* (2017.01)
*B01J 13/00* (2006.01)        *B01J 13/12* (2006.01)
*A61K 9/127* (2006.01)        *B01J 13/04* (2006.01)

(86) International application number:
**PCT/JP2010/070274**

(87) International publication number:
**WO 2011/062132 (26.05.2011 Gazette 2011/21)**

(54) **PROCESS FOR PRODUCTION OF W/O/W EMULSION, PROCESS FOR PRODUCTION OF LIPOSOME EMPLOYING THE PROCESS, AND POROUS MEMBRANE FOR USE IN THE METHODS**

VERFAHREN ZUR HERSTELLUNG EINER W/O/W-EMULSION, VERFAHREN ZUR HERSTELLUNG VON LIPOSOMEN UNTER ANWENDUNG DIESES VERFAHRENS UND PORÖSE MEMBRAN ZUR VERWENDUNG IN DIESEN BEIDEN VERFAHREN

PROCÉDÉ POUR LA PRODUCTION D'ÉMULSION W/O/W, PROCÉDÉ POUR LA PRODUCTION DE LIPOSOME UTILISANT LE PROCÉDÉ, ET MEMBRANE POREUSE POUR UTILISATION DANS LES PROCÉDÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2009 JP 2009265653**

(43) Date of publication of application:
**26.09.2012 Bulletin 2012/39**

(73) Proprietor: **Konica Minolta Holdings, Inc.**
**Tokyo 100-0005 (JP)**

(72) Inventors:
• **ISODA, Takeshi**
**Hino-shi**
**Tokyo 191-8511 (JP)**
• **MOTOKUI, Yasuyuki**
**Hino-shi**
**Tokyo 191-8511 (JP)**
• **WADA, Takeshi**
**Hino-shi**
**Tokyo 191-8511 (JP)**

(74) Representative: **Gille Hrabal**
**Brucknerstrasse 20**
**40593 Düsseldorf (DE)**

(56) References cited:
WO-A1-2005/053643        WO-A1-2009/142018
JP-A- 5 220 382           JP-A- 2000 190 416
JP-A- 2005 279 326        JP-A- 2009 084 293
US-A- 5 326 484           US-A1- 2006 220 269
US-A1- 2007 248 541

**Description**

TECHNICAL FIELD

The present invention relates to a process for producing liposomes that are used in the fields of medicines, cosmetics, foods, etc.

BACKGROUND ART

Liposomes are each a closed vesicle composed of a lipid bilayer membrane of a single layer or plural layers, and a liposome can hold, in its inner aqueous phase and inside the lipid bilayer membrane, water-soluble drugs and the like and hydrophobic drugs and the like, respectively. Moreover, since the lipid bilayer membrane of the liposomes is analogous to a biomembrane, the liposomes have high safety in vivo; therefore, various uses, such as medicines for DDS (drug delivery system), have been paid attention, and studies and development thereof have been promoted.

Although many processes for producing liposomes have been proposed, there are only few general-use techniques that can produce liposomes approvable as medicines and that are superior in costs and from the industrial viewpoint. Although a water-soluble drug can be contained in an inner aqueous phase of a liposome, even in the case of a conventional process for producing liposomes that is considered to be good, the drug encapsulation rate (the rate of weight of a drug or the like encapsulated in a liposome relative to the total weight of the drug or the like contained in a finally obtained liposome suspension (a liposome and an outer aqueous phase)) is about 40%, and, in most cases, an unencapsulated drug is wasted. Processes for producing liposomes that can provide a higher drug encapsulation rate, such as a reverse-phase evaporation method, are poor in reproducibility and are not applicable from the industrial viewpoint. Moreover, in recent years, nuclear acid medicines or the like have drawn public attention; however, since such medicines are unstable in vivo when used as a single material, carriers for use in stabilizing these have been demanded. For this reason, processes for producing liposomes have been demanded which can effectively encapsulate a water-soluble drug and which have an average particle diameter of a nano-size (preferably, 300 nm or less, which is suitable for phleboclysis).

As a process for producing liposomes having such a nano-size, various processes have been proposed so far. For example, patent literature 1 mentions a process for producing liposomes including a step in which, by allowing an aqueous solution containing a water-soluble drug to pass through a microchannel subjected to a hydrophilic surface treatment, the aqueous solution is dispersed in an oil phase containing an emulsifier, to give a W/O emulsion, and a step in which an oil-phase portion containing the emulsifier is removed in a frozen state of the W/O emulsion and substituted by an oil phase containing a lipid component, and then the oil-phase solvent is removed. However, in the process mentioned in patent literature 1, the encapsulation rate of the encapsulated substance encapsulated inside the liposomes thus produced is limited only to 6 to 45%.

Moreover, other known processes for producing liposomes include a process using a two-step emulsification method.

For example, non-patent literature 1 discloses a micro capsulation process in which a W/O/W emulsion emulsified by a two-step emulsification method is dried in a liquid, to form liposomes. Moreover, patent literature 2 mentions that, in a process for producing multivesicular liposomes that includes two-step emulsification processes, the encapsulation rate of a drug or the like can be improved by use of a lipid having a higher number of carbon atoms as a lipid component of liposomes. Furthermore, patent literature 3 mentions a process for producing multivesicular liposomes from a processed W/O/W emulsion, wherein the solvent is removed from the emulsion and a cross-flow filtering step is performed. Non patent literature 2 mentions that when a W/O/W emulsion is formed by using a microchannel emulsification method, with a W/O emulsion being used as a dispersion phase and a tris-hydrochloric acid buffer solution being used as an outer aqueous phase, sodium caseinate is introduced as an emulsifier into the outer aqueous phase, and thereby the encapsulation rate of calcein into a liposome (lipid capsule) can be enhanced to about 80%. However, these methods have a problem that it is difficult to obtain unilamellar liposomes having a uniform nano-size.

In recent years, with respect to the process for producing an emulsion by utilizing a two-step emulsification method, studies have been done which take into consideration the hydrophilic property and hydrophobic property of a microchannel substrate to be used, too. For example, patent literature 4 discloses an emulsion production device having three spaces separated by two kinds of microchannels having widths of 5 μm and 15 μm. In this emulsion production device, an area subjected to a hydrophobic surface treatment with a hydrophobic drug and an area subjected to a hydrophilic surface treatment with an oxygen plasma are combined with each other so that the inner wall and microchannels of the respective spaces get wet only with a continuous phase. Although this patent literature 4 mentions that a W/O/W emulsion has been produced by use of this emulsion production device, no specific descriptions are given regarding the use of this device for producing liposomes. Document US 5 326 484 discloses hydrophilic glass membranes for producing w/o/w emulsion, but does not disclose a method for producing liposomes.

Document US 2007/248541 discloses a method for producing liposomes. This method does not involve the use of porous membranes.

PATENT LITERATURE

Patent Literature 1: Japanese Patent No. 4009733
Patent Literature 2: JP-A No. 2001-505549
Patent Literature 3: JP-A No. 2001-522870
Patent Literature 4: JP-A No. 2003-71261
Patent Literature 5: JP-A No. S52-46382

NON PATENT LITERATURE

Non-Patent Literature 1: J Dispers Sci Technol, 9(1), 1-15 (1988)
Non-Patent Literature 2: Takashi Kuroiwa, Mitsutoshi Nakajima and Sosaku Ichikawa, "Influences of Aqueous Phase Composition on Lipid Capsule Formation Using Multiphase Emulsion as Base", Summary of the 74th Annual Meeting of the Society of Chemical Engineers, Japan (March, 2009)

SUMMARY OF INVENTION

PROBLEM TO BE SOLVED BY THE INVENTION

However, the above-mentioned prior-art techniques still have a problem in that it is still difficult to produce a W/O/W emulsion by use of an organic solvent having a low surface tension as an o-phase, and also to give a liposome that has both of properties of a high encapsulation rate of a water-soluble drug and a uniform nano-size. Therefore, an object of the present invention is to provide a process for producing liposomes, the process being able to provide unilamellar liposomes that have a particle diameter of a nano-size and a high encapsulation rate of a water-soluble drug.

In the present invention, the term "unilamellar liposome" (ULV), which has the same meaning as that of a mononuclear liposome, refers to a liposome structure having a single inner aqueous phase and the unilamellar liposomes usually have a volume mean particle diameter of about 20 to 500 nm. In contrast, the term "multivesicular liposome" (MVL) refers to a liposome structure containing a lipid membrane surrounding a plurality of non-concentric inner aqueous phases. Moreover, the term "multilamellar liposome (MLV)" refers to a liposome structure having a plurality of concentric membranes like "coats of onion", and shell-like concentric aqueous compartments in between the membranes. The feature of the multivesicular liposomes and the multilamellar liposomes is that they have a volume mean particle diameter in the range of micrometers and usually in the range of 0.5 to 25 $\mu$m.

When a W/O/W emulsion is produced from a W/O emulsion by use of a porous membrane, a system is used in which an oil phase containing the W/O emulsion and an outer aqueous phase are separated from each other with the porous membrane interposed therebetween. In this case, the W/O emulsion enters pores formed in the porous membrane by a pressure difference provided between the oil phase and the outer aqueous phase, and permeates through the porous membrane by passing through these pores. The W/O emulsion that has reached the outlet of each pore to be made in contact with the outer aqueous phase, is then allowed to form a droplet, and the droplet grows from the outlet of the pore toward the outer aqueous phase. As the droplet grows, the droplet is gradually necked at the vicinity of the outlet of the pore so that the droplet is finally separated from the porous membrane and dispersed into the outer aqueous phase. It is assumed that this phenomenon in which the droplet composed of the W/O emulsion is necked and separated from the porous membrane is caused by an interaction such as a surface tension and the like exerted among the droplet, the outer aqueous phase and the porous membrane surface. From this viewpoint, it is naturally predictable that in the case the O phase is an O phase composed of a solvent having a high surface tension, such as a sunflower oil, the solvent is easily necked to be separated from the porous membrane and that in the case the O phase is an O phase composed of an organic solvent having a low surface tension, this phenomenon tends to hardly occur. In fact, the aforementioned patent literature 4 mentions that a W/O/W emulsion was produced by use of a sunflower oil as its O phase. In addition, a patent literature 5 mentions that a W/O/W emulsion was produced by carrying out a stirring emulsification by use of light fluid paraffin as its O phase.

However, when a W/O/W emulsion is produced by use of a porous membrane such as a conventionally known microchannel substrate or the like, there is a problem that the particle diameter of the droplet may become so large due to continuous outflow of the W/O emulsion that it is not possible to form a W/O/W emulsion in which W/O emulsions having a predetermined particle diameter are dispersed. One reason for this is that since the hydrophilicity of a portion on the surface of the porous membrane made in contact with the outer aqueous phase is not sufficient, a droplet composed of the W/O emulsion is expanded along the surface of the porous membrane. Upon occurrence of this phenomenon, droplets to be discharged into outer aqueous phase become enormously big, or a layer made of the W/O emulsion is generated on the surface of the porous membrane on the portion made in contact with the outer aqueous phase because of joined droplets from the outlets of a plurality of pores mutually adjacent with one another, with the result that the droplet discharge into the outer aqueous phase might not occur in some cases.

Another point to be taken into consideration upon producing liposomes by formation of a W/O/W emulsion is that the solvent forming the O-phase needs to be removed from the resultant W/O/W emulsion. In this case, upon producing liposomes by using the process for producing a W/O/W emulsion, a volatile organic solvent tends to be used as a solvent forming the O-phase in that it is easily removed from the W/O/W emulsion. However, since volatile organic solvents generally have a low surface tension, the aforementioned problem caused by the continuous outflow of the W/O emulsion tends to become conspicuous. Although the problem caused by the continuous outflow of the W/O emulsion can be alleviated when a solvent having a high surface tension is used as the solvent forming the O-phase, the removing process from the W/O/W emulsion might be difficult since the solvent having a high surface tension generally tends to have a high boiling point. In relation to this point, the invention of the patent literature 4 describes a production of a W/O/W emulsion by the use of sunflower oil as a specific example of the O-phase; however, since the nonvolatile sunflower oil cannot be removed, conversion to liposomes is not available. Moreover, for the same reason as this, the use of a light flowing paraffin as the O-phase upon producing liposomes through formation of a W/O/W emulsion, as described in the patent literature 5, is not considered to be a practical solution, either.

If a sufficient hydrophilicity is applied to the porous membrane surface at the portion made in contact with the outer aqueous phase, that is, at least at the outlet of each pore and the periphery thereof on the porous membrane surface, the entire porous membrane at the portion made in contact with the outer aqueous phase is allowed to get wet with the outer aqueous phase; thus, the droplets made from the W/O/W emulsion can be easily separated from the porous membrane, without being expanded along the surface of the porous membrane. Moreover, if the hydrophilicity of the inside of each pore of the porous membrane is high, the interface between the outer aqueous phase and the droplet made of the W/O emulsion is easily allowed to reach the inside of each pore passing through the outlet of the pore so that the droplet made of the W/O emulsion is allowed to easily form a necked portion; thus, it is assumed that the droplet made of the W/O emulsion is further easily separated from the porous membrane.


MEANS TO SOLVE THE PROBLEM


The present inventors have been dedicated to making studies over and over and found out that the above-mentioned problems can be solved by producing liposomes via a W/O/W emulsion by using a porous membrane surface-treated with a hydrophilic drug so as to have a predetermined contact angle with water in the air, so that the present invention has been completed.

The current invention includes the following items:

[item 1] A process for producing liposomes comprising the following steps (1) to (3):

(1) a primary emulsification step of: emulsifying an organic solvent (O) that is a volatile organic solvent, an aqueous solvent (W1) and a mixed lipid component (F1), to give a W1/O emulsion;
(2) a secondary emulsification step of: dispersing the W1/O emulsion produced in the step (1) in an aqueous solvent (W2) by use of a porous membrane, to give the W1/O/W2 emulsion; and
(3) a solvent removal step of: removing the organic solvent (O) contained in the W1/O/W2 emulsion, to form a unilamellar liposome,

wherein the porous membrane has been surface-treated with a hydrophilic drug so as to allow its surface to have a contact angle with water in the air in a range from 0° or more to 42° or less,
wherein the hydrophilic drug is a silica precursor monomer or a silane coupling agent having a hydrophilic group on the surface thereof, the silane coupling agent containing at least one structure selected from the group consisting of a saccharide analog structure, an alicyclic polyol structure, a polyether structure, a polyamine structure and a quaternary ammonium structure, as the hydrophilic group,
wherein the mixed lipid component (F1) is at least one selected from the group consisting of a phospholipid, a sterol, a glycolipid, a glycol, an aliphatic amine and a longchain aliphatic acid,
wherein in the step (1), the organic solvent (O) is an organic solvent having a boiling point of less than 100°C and the emulsification is carried out with a substance to be encapsulated further contained in the aqueous solvent (W1),

wherein the step (2) is carried out with a dispersing agent further contained in the aqueous solvent (W2), and wherein the dispersing agent has a weight-average molecular weight of in a range of from 1, 000 to 100,000.

[item 2] The process according to [item 1], wherein the silane coupling agent contains at least one structure selected from the group consisting of a saccharide analog structure, an alicyclic polyol structure and a quaternary ammonium structure, as the hydrophilic group.

[item 3] The process according to [item 1], wherein the dispersing agent is at least one material selected from the group consisting of protein, polysaccharides and nonionic surface active agents.

[item 4] The process according to any one of [items 1 to 3], wherein the porous membrane is in the form of an SPG membrane or a microchannel substrate.

[item 5] The process according to [item 1], wherein in the step (1), the emulsification is carried out with a substance to be encapsulated further contained in the aqueous solvent (W1), and wherein a drug or the like for medical treatments is used as the substance to be encapsulated, and the resultant liposomes have a volume mean particle diameter of from 50 to 300 nm.

[item 6] The process for producing liposomes according to [item 1], further comprising a step of separating the dispersing agent and liposomes from each other.

Moreover, the organic solvent (O) is an organic solvent having a boiling point of less than 100°C from the viewpoint that after the preparation of the W1/O/W2 emulsion, the removal of the organic solvent (O) is easier.

In the present invention, the porous membrane is typically in the form of an SPG membrane or a microchannel substrate.

The process for producing liposomes in accordance with the present invention includes a step of removing the organic solvent (O) contained in the W1/O/W2 emulsion to form a unilamellar liposome.

In this case, preferably, a drug or the like for medical treatments is used as the substance to be encapsulated, and the resultant liposomes preferably have a volume mean particle diameter of from 50 to 300 nm.

In the present specification, "average particle diameter" refers to a volume mean particle diameter.

EFFECTS OF INVENTION

The production process of the present invention makes it possible to effectively produce liposomes each having an average particle diameter of a nano-size suitable for use in medicines and an excellent monodispersity. In particular, by use of the SPG membrane or microchannel substrate in the secondary emulsification step, the present process can be an industrially advantageous process having low costs and a high throughput. Moreover, in the case when the primary emulsification step is carried out, with a substance (in particular, a water-soluble drug or the like) to be encapsulated in liposomes being further added thereto, the present process can achieve an encapsulation rate higher than that of conventional technologies (for example, 80% or more), while imparting the above-mentioned characteristics to the liposomes. By utilizing the process for producing liposomes of the present invention, the productivity of liposomes suitable for various applications can be improved remarkably.

DESCRIPTION OF EMBODIMENTS

[Production Material]

Mixed lipid component (F1)

In the present invention, a mixed lipid component (F1) is used in the primary emulsification step. The mixed lipid component (F1) mainly forms a lipid bilayer membrane of liposomes.

The blending composition of the mixed lipid component (F1) is at least one selected from the group consisting of a phospholipid, a sterol, a glycolipid, a glycol, an aliphatic amine and a longchain aliphatic acid. Besides, it is not specifically restricted, but in general, the composition is mainly constituted of phospholipids (e.g., lecithin derived from animals and plants; phosphatidylcholine, phosphatidylserine, phosphatidylglycerol, phosphatidylinositol, glycerophospholipids that are phosphatidic acid or fatty acid esters thereof; sphingolipid; and derivatives thereof) and sterols that contribute to stabilization of lipid membrane (e.g., cholesterol, phytosterol, ergosterol, and derivatives thereof), and in addition, glycolipid, glycol, aliphatic amine, long-chain aliphatic acids (e.g., oleic acid, stearic acid, and palmitic acid), and other compounds that impart other various functions may be added. In the present invention, neutral phospholipids, such as dipalmitoyl phosphatidylcholine (DPPC), dioleyl phosphatidylcholine (DOPC) and dipalmitoyl phosphatidylglycerol (DPPG), are commonly used as the phospholipids. The blending ratio between the mixed lipid components is appropriately adjusted according to intended use, taking into consideration properties such as stability of the lipid membrane and behaviors of liposomes in vivo.

Moreover, as a specific example of the embodiment for carrying out the present invention, in addition to the mixed lipid component (F1), another mixed lipid component (F2) may be used in the secondary emulsification step, if necessary. The mixed lipid component (F2) mainly forms an outer membrane. The mixed lipid component (F1) and the mixed lipid component (F2) may have the same composition or mutually different compositions. As the components contained in the mixed lipid component (F2), the same components as those of the mixed lipid component (F1) may be used. Furthermore, by allowing the mixed lipid component (F2) to contain a lipid component required for adding functionalities as DDS, such as a PEG phospholipid, an effective modification may be carried out on the surface of liposome. The blending ratio between the mixed lipid components is also appropriately adjusted according to intended use, taking into consideration properties such as stability of the lipid membrane and behaviors of liposomes in vivo.

Aqueous Solvents (W1) and (W2), Organic Solvent (O)

As the aqueous solvents (W1) and (W2) and the organic solvent (O), publicly known general ones can be used. In the W1/O/W2 emulsion, the aqueous solvent (W1) and the aqueous solvent (W2) are solvents for use in forming the inner aqueous phase and the outer aqueous phase, respectively, and the organic solvent (O) is a solvent for use in forming an oil phase located in between the inner aqueous phase and the outer aqueous phase.

The aqueous solvents include aqueous solvents obtained by blending pure water with other compounds, such as salts or saccharides suitable for adjusting osmotic pressure, for adjusting pH, etc. Examples of the aqueous solvents include various buffer solutions, such as a tris-hydrochloric acid buffer solution. Moreover, in accordance with intended use of liposomes, materials, for example, gelling agents such as gelatin, thickener polysaccharides such as dextran, and charging polymers such as chitin/chitosan and poly-L-lysine, may be encapsulated in the aqueous solvent (W1).

The organic solvents include organic solvents incompatible with water, and examples thereof include: hexane (n-hexane: boiling point 69°C), pentane (boiling point 36°C), cyclohexane (boiling point 80.7°C), acetic acid esters (methyl acetate (boiling point 57°C), ethyl acetate (boiling point 77.1°C), isopropyl acetate (boiling point 89°C),, ethyl formate (boiling point 54°C), chloroform (boiling point 61.2°C), methylene chloride (boiling point 39.75°C), ethers (diethyl ether (boiling point 34.6°C), t-butylmethyl ether (boiling point 55°C)), 1,2-dichloroethene (cis form: boiling point 60°C, trans form: boiling point 48°C) and 1,1,2-trichloroethene (boiling point 87°C), and among these, hexane (n-hexane), pentane, cyclohexane, chloroform, methylene chloride, methyl acetate, ethyl acetate, isopropyl acetate, ethyl formate, diethyl ether and t-butylmethyl ether are preferably used. One kind of these organic solvents that are incompatible with water may be used alone, or two or more kinds of these may be used in a mixed form. Alternatively, one kind or two or more kinds of organic solvents that are easily compatible with water, such as acetonitrile (boiling point 81.6°C), ethanol (boiling point 78.3°C), methanol (boiling point 64.65°C), 2-propanol (boiling point 82.4°C), acetone (boiling point 56°C), methylethyl ketone (boiling point 80°C), THF (boiling point 66°C), 1,2-dimethoxyethane (boiling point 85°C) may be combined and used mixed with the organic solvent that is incompatible with water. As the organic solvent that is easily compatible with water, acetonitrile, ethanol, methanol, 2-propanol, acetone, methylethyl ketone, etc. are preferably used. Additionally, other ethers, hydrocarbons, halogenated hydrocarbons, halogenated ethers and esters than those described above, may also be used as the organic solvent (O). Moreover, a volatile organic solvent having a boiling point lower than that of water, that is, a volatile organic solvent having a boiling point less than 100°C, as the organic solvent (O) is used. The reason for this is because, by use of such an organic solvent (O), the organic solvent (O) can be easily removed in the step of producing liposomes from the W1/O/W2 emulsion.

Examples of more preferable modes of the mixed organic solvent include hexane:chloroform = 60:40 to 90:10, hexane:dichloromethane = 60:40 to 90:10, hexane:ethyl acetate = 50:50 to 90:10, and hexane:ethanol = 70:30 to 99:1 (each of these is represented by a volume ratio relative to 100 in total). As one of these modes of the mixed organic solvent, for example, a mixed organic solvent composed mainly of hexane (50% by volume or more) is preferably used because of its superior effect of improving the monodispersity of the resultant nano-size W1/O emulsion.

Additionally, as the organic solvent (O) to be used, three kinds or more of organic solvents may be combined with one another; however, from the viewpoint of easiness in setting conditions, two kinds of organic solvents are preferably combined with each other. If necessary, various kinds of functional components may be added to the mixed organic solvent.

Substances to be Encapsulated

In the present invention, the substances to be encapsulated in the emulsion or liposomes (referred to as "subject substance to be encapsulated") are not specifically restricted, and various substances that are known in the fields of medicines, cosmetics, foods, etc. (hereinafter, generically referred to as "drugs") can be used according to intended use of the liposomes.

Among these drugs, examples of water-soluble drugs for medical treatments include substances having pharmacological actions, such as contrast media (nonionic iodine compound for X-ray contrast radiography, complex composed of gadolinium and chelating agent for MRI contrast radiography, etc.), antitumor agents (adriamycin, pirarubicin, vincristine, taxol, cisplatin, mitomycin, 5-fluorouracil, irinotecan, estracyt, epirubicin, carboplatin, intron, Gemzar, methotrexate, cytarabine, Isovorin, tegafur, etoposide, Topotecin, pirarubicin, nedaplatin, cyclophosphamide, melphalan, ifosfamide, Tespamin, nimustine, ranimustine, dacarbazine, enocitabine, fludarabine, pentostatin, cladribine, daunomycin, aclarubicin, amrubicin, actinomycin, taxotere, trastuzumab, rituximab, gemtuzumab, lentinan, sizofiran, interferon, interleukin, asparaginase, fostestrol, busulfan, bortezomib, Alimta, bevacizumab, nelarabine, cetuximab, etc.), antibacterial agents (macrolide antibiotics, ketolide antibiotics, cephalosporin antibiotics, oxacephem antibiotics, penicillin antibiotics, $\beta$-lactamase agents, aminoglycoside antibiotics, tetracycline antibiotics, fosfomycin antibiotics, carbapenem antibiotics, penem antibiotics), MRSA·VRE·PRSP infectious disease remedies, polyene antifungal agents, pyrimidine antifungal agents, azole antifungal agents, candin antifungal agents, new quinolone synthetic antibacterial agents, antioxidants, anti-inflammatory agents, blood circulation accelerating agents, whitening agents, skin roughness preventing agents, anti-aging agents, hair growth accelerating agents, moisture retention agents, hormone agents, vitamins, nucleic acid (sense strand or anti-sense strand of DNA or RNA, plasmid, vector, mRNA, siRNA, etc.), proteins (enzyme, antibody, peptide, etc.), and vaccine formulations (those having toxides of tetanus and the like as antigens; those having viruses of diphtheria, Japanese encephalitis, poliomyelitis, rubella, mumps, hepatitis and the like as antigens; DNA or RNA vaccine; etc.), and formulation additives such as dyes/fluorescent dyes, chelating agents, stabilizers and preservatives.

The objective for allowing a water-soluble drug to be encapsulated can be achieved by dissolving the water-soluble drug in the inner aqueous phase (W1). Therefore, in the case when a highly water-soluble drug or the like is encapsulated as the subject substance to be encapsulated, by dissolving the drug in the inner aqueous phase (W1) with a high concentration, the absolute amount of the drug to be encapsulated in the emulsion or liposomes can be increased. On the other hand, the amount of the inner aqueous phase (W1) can be changed on demand, and in an attempt to form particles (W1/O) having a predetermined particle diameter, the amount (number of pieces) of the lipids required for this purpose can be calculated. For example, in the case when a W1/O emulsion of 100 nm (particle volume: $0.0005 \ \mu m^3$) is formed, the amount of W1/O particles to be generated upon production by using 1.0 mL of the W1 phase (inner aqueous phase) can be calculated as $2.0 \times 10^{15}$ pieces. On the other hand, when the surface area of the W1/O emulsion is taken into consideration, in the case lecithin is used as phospholipids, supposing that the surface area per molecule of lecithin is $0.7 \ nm^2$, the number of the phospholipid molecules forming the W1/O nano-emulsion of 100 nm (particle surface area 4n $\times$ 2500 $nm^2$) is calculated as $0.4 \times 10^5$ pieces. Therefore, the amount of lipids required for a primary emulsification of 1.0 mL of the drug corresponds to ($2.0 \times 10^{15}$ pieces) $\times$ ($0.4 \times 10^5$ pieces) = $0.8 \times 10^{20}$ pieces, that is, 0.132 mmols. With respect to lipid molecules other than lecithin, the surface area thereof may also be considered as about $0.7 \ nm^2$; therefore, the total amount of lipids of 0.132 mmols is considered to be the minimum amount required for forming the W1/O nano-emulsion of 100 nm. In an attempt to form liposomes, an amount of 0.264 mmols, which is twice as much, is required, and when molecular-weight converted by DPPC that is a typical phospholipid, an amount of 193 mg is required.

In the case when a drug is dissolved into 1.0 mL of water and liposomes of 100 nm are formed, since 0.1 to 1.0 g of cytarabine is dissolved in 1.0 mL of water as defined in the Pharmacopoeia, the drug weight ratio is from 0.1 g/0.193 g to 1.0 g/0.193 g, and since 1.0 g or more iohexol (contrast medium) is dissolved in 1.0 mL of water, the drug weight ratio is 1.0 g/0.193 g or more. In this case, the drug weight ratio represents a weight ratio between the drug to be used for forming liposomes, and phospholipid. This high drug weight ratio means that by reducing the amount of lipid, the drug can be efficiently encapsulated, and the fact that the amount of lipid can be reduced is significant from the medical viewpoint. In this manner, the drug weight ratio of 0.5 to 5 can be achieved by the method of the present invention. In general, when more drug is dissolved, the viscosity becomes higher, with the solubility coming closer to a saturated state. By use of the method of the present invention, the drug can be encapsulated up to a viscosity of 10 mPa·s in the inner aqueous phase.

Moreover, in the case when liposome particles larger than 100 nm are produced, since the required amount of lipid can be reduced, a more effective process can be achieved.

Dispersing Agent

In the present invention, for the purpose of stabilizing an emulsion and liposomes to be produced, an outer aqueous phase containing a dispersing agent is used in a secondary emulsification step, if necessary. In this case, the secondary emulsification step of the present invention is carried out, with the dispersing agent being further contained in the aqueous solvent (W2).

dispersing agents preferably used in the present invention typically include dispersing agents which form no molecular self-aggregate or dispersing agents which exclusively form molecular self-aggregates having a volume mean particle diameter of not more than 10 nm (such dispersing agents may be referred to as "specific dispersing agent") . When such a specific dispersing agent is used as the dispersing agent, a desirable process is provided in separating liposomes and the dispersing agent from each other in a filtering step. In a preferable mode in the present invention, an outer aqueous phase containing this specific dispersing agent is used in the secondary emulsification step.

In the present specification, the outer aqueous phase containing a "dispersing agent which forms no molecular self-aggregate" indicates both a case where a substance which forms no molecular aggregate (typically, micelles) even if the concentration is increased is added to the outer aqueous phase as a dispersing agent, and a case where a substance which forms molecular aggregates in a certain concentration or higher (typically, surface active agent having a critical micelle concentration) is added as a dispersing agent to the outer aqueous phase in a concentration lower than said certain concentration. Moreover, the outer aqueous phase containing a "dispersing agent which exclusively forms molecular self-aggregates having a volume mean particle diameter of not more than 10 nm" indicates a case where a substance which may form molecular aggregates whose volume mean particle diameter is not more than 10 nm is added as a "dispersing agent" to the outer aqueous phase.

It is considered that the dispersing agents may be roughly classified into two types from the viewpoint of function. Dispersing agents of one type are those which are distributed all over the outer aqueous phase (W2) because of relatively low orientation to the interface between the W1/O emulsion and the outer aqueous phase (W2) and have a function to stabilize liposomes by preventing W1/O/W2 emulsion from sticking one another, such as polysaccharides. Dispersing agents of the other type are those which have relatively high orientation to the interface of the W1/O/W2 emulsion and have a function to stabilize liposomes by surrounding the emulsion like a protective colloid, such as protein and nonionic surface active agents.

If W1/O/W2 emulsions coalesce with one another to form particles of larger diameters, removal of solvent by in-liquid drying method, etc. is non-uniformly carried out, the encapsulated drug is liable to leak, etc., that is, liposomes become unstable; however, the dispersing agent makes it possible to prevent such destabilization, and contributes to enhancement of efficiency in the formation of unilamellar liposomes and to enhancement of the encapsulation rate of the drug. If the dispersing agent is orientated to the interface of the W1/O/W2 emulsion, individual liposomes tend to get untied when the liposomes are formed with removal of the solvent, so that the dispersing agent also contributes to enhancement of efficiency in the formation of unilamellar liposomes and to enhancement of the encapsulation rate of the drug. It is assumed that since the patent literature 2 and patent literature 3 do not use such a dispersing agent having these effects, it is not possible to obtain unilamellar liposomes having a uniform nano-size, and multivesicular liposomes are obtained.

Additionally, upon formation of liposomes, the dispersing agent exerts predetermined functions as described above, and since the mixed lipid component, constituted mainly of phospholipids having hydration ability, has a self-organizing capability, the dispersed state can be maintained even if no dispersing agent is present after the formation of liposomes.

Typical examples of the dispersing agent to be used in the present invention include protein, polysaccharides and nonionic surface active agents, but the dispersing agent is not limited to these examples, and other substances having a given function as dispersing agent may be used given that the dispersing agent has a weight-average molecular weight of in a range of from 1,000 to 100,000. When the dispersing agent is a substance approved as an additive in medicines, etc. (substance guaranteed to have no serious influence on the human body even if it is administered into the body), there is no substantial clinical problem even if a part of it remains in the liposome dispersion after a filtration step.

Examples of the proteins include gelatin (soluble protein obtained by denaturing collagen through heating), albumin. Gelatin usually has a molecular weight distribution from several thousands to several millions, and for example, gelatin having a weight-average molecular weight of 5,000 to 100,000 is preferable. Gelatin commercially available as that for medical treatments or foods can be used. Examples of the albumin include egg albumin (molecular weight: about 45,000), serum albumin (molecular weight: about 66,000... bovine serum albumin) and lactalbumin (molecular weight: about 14,000 ... $\alpha$-lactalbumin), and for example, dry desugared albumen that is egg albumin is preferable.

Examples of the polysaccharides include dextran, starch, glycogen, agarose, pectin, chitosan, carboxylmethyl cellulose sodium, xanthan gum, locust beam gum and guar gum, and the like, for example, dextran having a weight-average molecular weight of 10,000 to 100,000 is preferable.

The nonionic surface active agent is a surface active agent having a hydroxyl group and an ether bond that are not dissociated in water as a hydrophilic group. The nonionic surface active agents are roughly classified into a polyalkylene glycol type and a polyhydric alcohol type, and typical examples of the former include products, such as "Pluronic F-68" (available from BASF, polyoxyethylene (160) polyoxypropylene (30) glycol, number-average molecular weight: 9600), and polyethylene glycols having a weight-average molecular weight of 1000 to 8000. The Pluronic-type nonionic surface active agents developed as copolymers between polyethylene glycol and polypropylene glycol have many kinds of products depending on their degree of polymerization, and they are generically referred to as Pluronics.

Typical examples of the former include: "Tween 80" (available from TOKYO CHEMICAL INDUSTRY CO. LTD., polyoxyethylene sorbitan monooleate, molecular weight: 1309.68), which is a surface active agent obtained by allowing the same polyalkylene glycol-based compound as described above to react with a compound having a hydroxyl group, such as glycerin and sorbitan. With respect to this, many kinds of products have also been known depending on their degree of polymerization.

Whether the dispersing agent forms molecular self-aggregates in the outer aqueous phase or not, or whether the volume mean particle diameter of the molecular aggregates formed is not more than 10 nm or not (that is, whether the substance added to the outer aqueous phase satisfies requirements for the specific dispersing agent or not) can be confirmed by, for example, a particle size distribution meter of dynamic light scattering type or a freeze fracturing method using a transmission electron microscope (TEM).

The amount of the dispersing agent added to the outer aqueous phase (concentration of the dispersing agent) may be adjusted in an appropriate range according to the type of the dispersing agent. When a substance which forms molecular self-aggregates (having a volume mean particle diameter of more than 10 nm) in a certain concentration is added as the dispersing agent, the amount added is adjusted in a range wherein the concentration is less than the certain concentration. Too high concentration may bring troubles to the measurement by the particle size distribution meter depending upon the type of the dispersing agent, and therefore, it is preferable to adjust the concentration in a range of low concentrations that would not bring such troubles. For example, in the case when the above-mentioned Pluronic F-68, casein or the like is used as the dispersing agent, the concentration of the dispersing agent in the aqueous solvent (W2) to be used as the outer aqueous phase is desirably set to 0.0001 to 5%, preferably, to 0.001 to 0.5%, and more preferably, to 0.01 to 0.1%.

Taking into consideration separation between liposomes and the dispersing agent in the filtration step, the volume mean particle diameter of the molecular self-aggregates of the dispersing agent or assemblies thereof is preferably set to not more than 1/10, more preferably not more than 1/100, of the volume mean particle diameter of the liposomes.

If the molecular weight of the dispersing agent is too low, the dispersing agent may be liable to be incorporated into the lipid membrane to inhibit formation of liposomes; in contrast, if the molecular weight thereof is too high, the rate of dispersion of the W1/O/W2 emulsion in the outer aqueous phase or the rate of orientation to the interface may be lowered to cause coalescence of liposomes or formation of multivesicular liposomes. On this account, the weight-average molecular weight of the dispersing agent is in a range of from 1,000 to 100,000.

[Porous Membrane]

A porous membrane to be used in the present invention is used for the purpose of dispersing a W1/O emulsion into an aqueous solvent (W2) so that the W1/O/W2 emulsion is formed, in a secondary emulsification step of the process for producing a W1/O/W2 emulsion according to the present invention. The porous membrane in the present invention is provided with a surface that is allowed to easily get wet with the aqueous solvent (W2) so that droplets made from the W1/O emulsion can be surely separated from the porous membrane without being expanded along the surface of the porous membrane, and dispersed in the aqueous solvent (W2).

More specifically, the porous membrane of the present invention is subjected to a surface treatment with a hydrophilic drug so as to allow the surface to have a contact angle with water in the air in a range from 0° or more to 42° or less. The porous membrane of the present invention is typically provided with a porous membrane forming a raw material (hereinafter, referred to as "base porous membrane") and a hydrophilic surface treated layer derived from the hydrophilic drug covering the surface of the base porous membrane, and also has the structure that makes a contact angle of the surface with water in the air in a range from 0° or more to 42° or less.

The smaller the contact angle with water in the air is, the more easily the porous membrane of the present invention gets wet with the aqueous solvent (W2), and, usually, the more hardly it gets wet with the organic solvent (O). For this reason, the smaller the contact angle with water in the air is, the more easily the droplets made of the W1/O emulsion are separated from the porous membrane without being expanded along the surface of the porous membrane, and the more easily they are dispersed in the aqueous solvent (W2). For this reason, the porous membrane to be used in the present invention is a porous membrane that has been subjected to a surface treatment with a hydrophilic drug so as to allow the surface to have a contact angle with water in the air in a range from 0° or more to 42° or less, preferably from 0° to 40°, and more preferably, from 0° to 35°.

Moreover, the porous membrane of the present invention, which is used for dispersing the W1/O emulsion in the aqueous solvent (W2) so as to form a W1/O/W2 emulsion, tends to have time-related degradation in its hydrophilicity depending on the material and structure of the surface of the porous membrane, since various components contained in the W1/O emulsion gradually adhere to the surface during the use, even when it had a sufficient hydrophilicity prior to the use. For example, in the case when a microchannel made of silicon that has been subjected only to a surface treatment with plasma is used as the porous membrane, the contact angle with water in the air is about 30° immediately after an acid treatment; however, when this is used for forming a W1/O/W2 emulsion, the contact angle tends to increase to about 70 to 80° in about 3 hours due to adhesion of stains. Moreover, in the case when a porous membrane made of porous glass, such as a loam porous glass membrane, is used as the porous membrane, the contact angle with water in the air is about 25° immediately after an acid treatment; however, the contact angle tends to increase to about 80° as adhesion of stains develops during the use. Such an increase in the contact angle due to adhesion of stains tends to be caused even when the membrane is merely immersed in tap water. Upon occurrence of such a phenomenon, the porous membrane is hardly made to get wet with the aqueous solvent (W2), and the droplets made of the W1/O emulsion tend to be easily expanded along the surface of the porous membrane, with the result that the droplets are hardly separated from the porous membrane into the aqueous solvent (W2). Taking this fact into consideration, the porous membrane to be used in the present invention is preferably subjected to a surface treatment such that not only the contact angle with water in the air prior to use, but also the contact angle with water after the use for a predetermined period of time (about several weeks) is adjusted to be the aforementioned angle. In other words, the porous membrane to be desirably used in the present invention is a porous membrane that is subjected to a surface treatment such that not only the contact angle with water in the air prior to use, but also the contact angle with water in the air after the use, is adjusted in a range from 0° or more to 42° or less, preferably, from 0° to 40°, and more preferably, from 0° to 35°.

In the present invention, the "contact angle" can be measured and evaluated by carrying out measurements using a method such as a $\theta/2$ method on an equivalent surface treated substrate obtained when the surface treatment using the same hydrophilic drug as that used for forming the porous membrane to be actually used is carried out onto a plane substrate having the same material as that of the porous membrane to be actually used for forming a W1/O/W2 emulsion, that is, onto a substrate having the same material as that of the base porous membrane forming the porous membrane to be actually used.

With respect to a porous membrane to be used in the present invention, although its mode is not particularly limited, examples of the typical mode include a loam porous glass membrane (hereinafter, referred to as "SPG membrane") or a microchannel substrate.

Moreover, the size of each pore in the porous membrane of the present invention can be adjusted on demand in accordance with the size of a W1/O/W2 emulsion to be formed. For example, in the case when the microchannel substrate is used as the porous membrane, the terrace length, channel depth and channel width of the microchannel substrate can be adjusted on demand, and in the case when the SPG membrane is used as the porous membrane, the fine pore diameter of the SPG membrane can also be adjusted on demand, in accordance with the size of the W1/O/W2 emulsion to be respectively formed. For example, the fine pore diameter of the SPG membrane is typically adjusted to 0.1 to 100 $\mu$m.

Base Porous Membrane

In the present specification, "base porous membrane" is a porous membrane forming a material of the porous membrane of the present invention, that is, a porous membrane serving as a base material of the porous membrane of the present invention. This base porous membrane forms a skeleton structure of the porous membrane of the present invention. A porous membrane that can be used as the base porous membrane of the present invention is not particularly limited, as long as it has a predetermined size of each pore and it does not lose its functions as a porous membrane even when surface-treated with a hydrophilic drug. However, from the viewpoint of wide use in the conventionally known process for producing a W1/O/W2 emulsion, the base porous membranes to be used in the present invention typically include porous glass membranes, such as a loam porous glass membrane (hereinafter, referred to as "SPG membrane"), as well as microchannel substrates formed by a material, such as silicon, resin, metal. Examples of the resin which can be used as the microchannel substrate include: thermoplastic resins, such as acryl-based resins, cycloolefin-based resins, styrene-based resins, acryl-styrene-based copolymers, polycarbonate-based resins, polyester-based resins such as polyethylene terephthalate, ethylene-vinyl alcohol copolymers, thermoplastic elastomers, such as those of olefin-based, styrene-based and urethane-based, vinyl chloride-based resins, fluorine-based resins, and silicone-based resins such as polydimethyl siloxane; thermosetting resins, such as epoxy-based resins, phenol-based resins, urethane-based resins and unsaturated polyester-based resins; and photocurable resins, such as multifunctional acryl-based resins. The metals which can be used for the microchannel substrate include: nickel, platinum, silver, gold, copper, aluminum, etc.

Hydrophilic Drug

In the present invention, "hydrophilic drug" is used for improving the hydrophilicity of the surface of a porous membrane.
The hydrophilic drug is a silica precursor monomer or a silane coupling agent having a hydrophilic group on the surface thereof, the silane coupling agent containing at least one structure selected from the group consisting of a saccharide analog structure, an alicyclic polyol structure, a polyether structure, a polyamine structure and a quaternary ammonium structure, as the hydrophilic group. The "hydrophilic drug" is a drug that forms, on the porous membrane, a hydrophilic surface having a contact angle with water in the air in a range from 0° or more to 42° or less, preferably, from 0° to 40°, and more preferably, from 0° to 35°, when the base porous membrane is subjected to a surface treatment.

Moreover, the "hydrophilic drug" is more preferably a drug that can form on the porous membrane a hydrophilic surface whose hydrophilicity is less susceptible to time-related degradation during use, and the drug preferably makes it possible to form a porous membrane that can maintain the contact angle of the surface with water in the air in the above-mentioned range, that is, from 0° or more to 42° or less, preferably, from 0° to 40°, and more preferably, from 0° to 35°, even after a lapse of about several weeks.

Moreover, the porous membrane to be used in the present invention is preferably designed so as not to lose its hydrophilicity acquired by a surface treatment under conditions in which the emulsification step of W1/O emulsion is carried out. For this reason, the hydrophilic drug desirably are hydrophilic molecules having such a functional group as to form a chemical bond with a functional group on the surface of the base porous membrane, or are drugs that form an insoluble coat membrane having a hydrophilic structure on the surface of the base porous membrane. Such a hydrophilic drug may be either a drug having both of a hydrophilic structure and such a structure as to be bonded to the base porous membrane surface in one molecule, or a combination of a drug having a hydrophilic structure and a drug having the structure to be bonded to the base porous membrane surface.

Typical examples of the hydrophilic molecule having such a functional group as to form a chemical bond with a functional group on the base porous membrane surface (hereinafter, referred to also as "hydrophilic molecule") include silane coupling agents having a hydrophilic group on the surface thereof. In a preferable mode carried out in the present invention, as such a silane coupling agent, a silane coupling agent which is provided with at least either one of a nonionic hydrophilic group and a cationic hydrophilic group is proposed; that is, more specifically, a silane coupling agent, which has at least one structure selected from the group consisting of a polyol structure, a polyether structure, a polyamine structure and a quaternary ammonium structure, as the hydrophilic group, is proposed. Among these, the polyol structure and the polyether structure are included in the nonionic hydrophilic group. Moreover, each of the polyamine structure and the quaternary ammonium structure functions as a cationic hydrophilic group when allowed to have the corresponding ammonium-type structure. Examples of such silane coupling agents include a silane coupling agent having a saccharide analog structure typically represented by N-(3-triethoxysilylpropyl)glconamide; silane coupling agents having a polyethylene glycol structure; silane coupling agents having a polyethyleneimine structure typically represented by trimethoxysilylpropyl modified polyethyleneimine commercially available from Gelest, Inc. as catalog number SSP-060; and silane coupling agents having a lower hydroxyalkylamine structure such as a diethanolamino group; however, the present invention is not intended to be limited by these.

In a typical mode carried out in the present invention, the "silane coupling agent having a hydrophilic group on its surface" is provided with a hydrophilic portion containing the hydrophilic group and a reactive functional group portion containing a silicon-based functional group capable of being bonded to the functional group that is present on the base porous membrane surface, and has a structure in which the hydrophilic portion and the reactive functional group portion are directly bonded to each other, or indirectly bonded to each other through a spacer portion having an appropriate chain length.

In this case, the "silane coupling agent having a saccharide analog structure" is proposed as a preferable example of "silane coupling agent having a polyol structure". In this "silane coupling agent having a saccharide analog structure", a polyol portion derived from sugar functions as a hydrophilic group. Preferable examples of these "silane coupling agents having a saccharide analog structure" include those agents having a structure in which a carboxyl group forming a carboxylic acid obtained by oxidizing sugar is bonded to the reactive functional group portion or the spacer portion through an amide bond or an ester bond, and examples of the "carboxylic acid obtained by oxidizing sugar" to be used at this time include an aldonic acid such as a gluconic acid and an uronic acid such as a glucuronic acid. However, the "silane coupling agents having a saccharide analog structure" are not limited to these, and those silane coupling agents having a structure in which the polyol portion derived from sugar and the reactive functional group portion or the spacer portion are bonded to each other through a bond of another kind, such as a carbon-carbon bond, may also be used. Moreover, as long as the hydrophilicity is not impaired, the formation of a W/O/W emulsion is not disturbed, or no loss of the substance to be encapsulated is caused, some of hydroxyl groups forming the polyol portion derived from sugar may be modified, or may be substituted by another functional group. Sugars forming the skeleton of the polyol portion are not limited to monosaccharides, as long as the hydrophilicity of the entire polyol portion is not impaired, and may be di- or higher saccharides, for example, oligosaccharides. As mentioned above, as a preferable example of the "silane coupling agent having a polyol structure", the "silane coupling agent having a saccharide analog structure" is proposed; however, silane coupling agents having a polyol structure derived from a polyol other than a saccharide analog, such as an alicyclic polyol, may be used as the "silane coupling agent having a polyol structure".

With respect to the "silane coupling agent having a cationic hydrophilic group", the following description will additionally explain the tertiary amine structure, which are not according to the current invention, and the quaternary ammonium structure given as examples of the hydrophilic group functioning as a cationic hydrophilic group. In amines of the tertiary or less, which are not according to the current invention, those having a free amine type structure and those having an ammonium type structure corresponding to covalent acids thereof are present at a certain ratio by the acid-base balance. In this case, the tertiary amine structure functions as a cationic hydrophilic group by taking the corresponding ammonium type structure. However, the proportion of amines having the ammonium type structure in the entire amines may vary depending on the liquid properties of a solvent as well as on the electronic characteristic and hydrophilicity of a substituent bonded to the central nitrogen atom. The same is true for a polyamine structure. On the other hand, since the quaternary ammonium salt has all the four hydrogen atoms forming ammonium ($NH_4^+$) in the narrow sense substituted by substituents such as alkyl groups, it is allowed to maintain the ammonium type structure regardless of liquid characteristics of a solvent located on the periphery thereof under conditions of forming a normal W/O/W emulsion. From this point of view, of the tertiary amine structure, which is not according to the current invention, and the quaternary ammonium structure given as the examples of the hydrophilic group in the "silane coupling agent having a hydrophilic group on its surface", the quaternary ammonium structure is particularly preferable. However, since the hydrophilicity exerted by the ammonium type structure is also influenced by a substituent to be bonded to the central nitrogen atom, it is preferable not to allow such a functional group as to impair the hydrophilicity to exist as the substituent to be bonded to the central nitrogen atom as long as it is possible, except for a functional group required for exerting functions as the silane coupling agent. From this point of view, specifically preferable examples of the silane coupling agent having a cationic hydrophilic group include silane coupling agents which have a quaternary ammonium structure, wherein one of substituents bonded to the central nitrogen atom is an alkyl group having a reactive functional group portion including a silicon-based functional group capable of being bonded to a functional group located on the base porous membrane surface, and the rest three substituents are lower alkyl groups such as a methyl group and an ethyl group.

Such hydrophilic molecules may be formed by copolymerizing a silane coupling agent having polymerizable unsaturated double bonds with a hydrophilic compound having polymerizable unsaturated double bonds, and for example, prepared as hydrophilic molecules of a copolymer type obtained by copolymerizing a (meth)acrylate-based silane coupling agent such as (3-trimethoxysilylpropyl) methacrylate or a styrene-based silane coupling agent such as p-(trimethoxysilyl)styrene, with a monomer having a hydrophilic group such as a (meth) acrylate monomer having a polyether group (for example, methoxypolyethylene glycol-methacrylate commercially available as BLEMMER (registered trademark) PME-400 from NOF CORPORATION).

The hydrophilic group contained in the silane coupling agent is at least either one of a nonionic hydrophilic group and a cationic hydrophilic group, and has at least any one of the structures selected from the group consisting of a saccharide analog structure, an alicyclic polyol structure, a polyether structure, a polyamine structure and a quaternary ammonium structure.

Use of such a hydrophilic molecule as a hydrophilic drug is advantageous in making it possible to provide, as the porous membrane of the present invention, a porous membrane having pores that have virtually the same size as that of the pores formed in the base porous membrane, since a hydrophilic surface treated layer is easily formed on the base porous membrane surface in the form of a mono-molecular layer. These hydrophilic molecules may be used alone or in combination of two or more kinds.

Moreover, the drugs that form an insoluble coat membrane having a hydrophilic structure on the surface of the base porous membrane (hereinafter, referred to also as "hydrophilic coat membrane forming agent") include compounds capable of forming a layer made from a hydrophilic inorganic compound such as silica on the base porous membrane surface. In the present invention, as such a compound, from the viewpoints of chemical stability and of preventing the hydrophilic structure formed on the base porous membrane surface from denaturing components forming a W1/O emulsion, compounds capable of forming a hydrophilic layer made from silica are typically used. Examples of the compounds include silica precursor monomers such as tetraalkylsilane, halogenated silicon and polysilazane. Examples of tetraalkyl silane include a lower alkyl silane, such as tetraethoxysilane.

These hydrophilic coat membrane forming agents may be used alone or in combination of two or more kinds.

Use of such a hydrophilic coat membrane forming agent as the hydrophilic drug is advantageous in forming a hydrophilic treated surface layer having high stability on the base porous membrane. However, since the thickness of the hydrophilic treated surface layer formed on the surface of each of pores formed in the base porous membrane may be greater depending on conditions of forming the insoluble coat membrane, it may be necessary to set the size of the pores to be preliminarily formed in the base porous membrane greater so as to allow pores in the resultant porous membrane to have a predetermined size, by taking into consideration a contracted portion of each pore caused depending on the thickness of the hydrophilic treated surface layer.

As a hydrophilic drug to be used in the present invention, either one of the "hydrophilic molecules" and the "hydrophilic coat membrane forming agent" may be used, or the two of these may be used in combination.

Surface Treatment

A porous membrane to be used in the present invention can be obtained by carrying out a surface treatment on demand with the hydrophilic drug on the base porous membrane so as to make the contact angle of its surface with water in the air at a predetermined angle. For example, the porous membrane in the form of an SPG membrane or a microchannel substrate can be respectively obtained by subjecting a material SPG membrane or a material microchannel substrate forming a base porous membrane to a surface treatment with a hydrophobic drug.

For example, in the case when the "hydrophilic molecules" are used as the hydrophilic drug, the surface treatment can be carried out through processes in which, after, if necessary, introducing a reactive functional group into the base porous membrane with a plasma treatment or the like, the "hydrophilic molecules" diluted, as necessary, into an appropriate solvent such as ethanol are allowed to react with the base porous membrane.

In the case when the hydrophilic molecules are formed by copolymerizing a silane coupling agent having polymerizable unsaturated double bonds with a hydrophilic compound having polymerizable unsaturated double bonds, the surface treatment can be carried out by first (i) introducing a reactive functional group into the base porous membrane with a plasma treatment or the like, if necessary, and (ii) introducing an unsaturated double bonding functional group into the base porous membrane by allowing the "silane coupling agent having polymerizable unsaturated double bonds" diluted, as necessary, in an appropriate solvent such as ethanol or the like to react with the base porous membrane, and then (iii) further introducing a hydrophilic functional group into the base porous membrane by copolymerizing with the "hydrophilic compound having polymerizable unsaturated double bonds".

In the case when the "hydrophilic coat membrane forming agent" is used as the hydrophilic drug, the surface treatment can be carried out through processes in which, after, if necessary, introducing a reactive functional group into the base porous membrane by a plasma treatment or the like, the base porous membrane is directly processed with the "hydrophilic coat membrane forming agent". Moreover, the surface treatment with the "hydrophilic coat membrane forming agent" may be carried out through processes in which, after an appropriate binder layer has been preliminarily formed on the surface of the base porous membrane, the binder layer is subjected to a surface treatment with the "hydrophilic coat membrane forming agent".

[Process for Producing W1/O/W2 Emulsion]

The process for producing a W1/O/W2 emulsion which is part of the process for producing liposomes according to the present invention is provided with the following steps (1) and (2).

(1) Primary emulsification step

A primary emulsification step is a step for preparing a W1/O emulsion by emulsifying the organic solvent (O), the aqueous solvent (W1) and the mixed lipid component (F1).

The preparation method for the W1/O emulsion is not particularly limited, and the preparation can be carried out by the use of apparatuses, such as ultrasonic emulsifier, stirring emulsifier, membrane emulsifier and high-pressure homogenizer. For the membrane emulsification, a premix membrane emulsification method wherein a W1/O emulsion of large particle diameters is prepared in advance and then the emulsion is passed through a membrane of small pore diameters to prepare a W1/O emulsion of smaller particle diameters may be used.

The pH value of the aqueous solvent (W1) is usually in the range of 3 to 10, and can be adjusted in a preferred range according to the mixed lipid component. For example, when oleic acid is used as the mixed lipid component, the pH thereof is preferably in the range of 6 to 8.5. For the pH adjustment, an appropriate buffer solution may be used.

The mean particle diameter of the W1/O emulsion, the proportion of the mixed lipid component (F1) added to the organic solvent (O), the volume ratio between the organic solvent (O) and the aqueous solvent (W1), and other operation conditions in the primary emulsification step can be properly controlled according to the emulsification method adopted, taking into consideration conditions of the succeeding secondary emulsification step, the type of the finally prepared liposomes, etc. In usual, the proportion of the mixed lipid component (F1) to the organic solvent (O) is in the range of 1 to 50% by mass, and the volume ratio between the organic solvent (O) and the aqueous solvent (W1) is in the range of 100:1 to 1:2.

In the present invention, in order to encapsulate a water-soluble drug or the like in liposomes, any of (i) a method in which a water-soluble drug or the like is dissolved or suspended in the aqueous solvent (W1) of the primary emulsification step in advance, and at the time of completion of the secondary emulsification step, liposomes encapsulating the drug or the like are obtained, and (ii) a method in which (empty) liposomes encapsulating no water-soluble drug or the like is first obtained, then a water-soluble drug or the like is added to the dispersion of the liposomes, or when a freeze-dried powder once obtained is redispersed in an aqueous solvent, a water-soluble drug or the like is added, and the mixture is stirred to incorporate the drug or the like into the liposomes may be used. Fat-soluble drugs or the like can also be encapsulated in the liposomes by adding them in advance in the primary emulsification step as in the method (i) or by adding them after obtaining empty liposomes as in the method (ii).

(2) Secondary emulsification step

The secondary emulsification step is a step to prepare a W1/O/W2 emulsion by dispersing the W1/O emulsion produced in the step (1) in the aqueous solvent (W2), by use of the porous membrane.

An outer aqueous phase containing a dispersing agent is preferably used in this secondary emulsification step. In this case, usually, an aqueous solvent (W2) for forming the outer aqueous phase and the dispersing agent are mixed to preliminarily prepare an outer aqueous phase in advance, and the W1/O emulsion is dispersed therein.

The method to prepare the W1/O/W2 emulsion in the secondary emulsification step includes a membrane emulsification method (microchannel emulsification method, emulsification method using SPG membrane, or the like) using the above-mentioned porous membrane. In particular, since the microchannel emulsification method and the emulsification method using SPG membrane do not require any great mechanical shearing force in the emulsification step, these methods are advantageous from the viewpoint of suppressing collapse of droplets and leakage of the encapsulated substance from the droplets at the time of the emulsifying operation.

In the membrane emulsification method, a premix membrane emulsification method (membrane permeation method), wherein a W1/O/W2 emulsion of large particle diameters is prepared in advance and then the emulsion is passed through a membrane of small pore diameters to prepare a W1/O/W2 emulsion of smaller particle diameters, may be used. The premix membrane emulsification method is preferable because energy required is low, the throughput is large, and preparation of liposomes can be sped up.

The mode of mixing (order of addition, or the like) of the aqueous solvent (W2), the W1/O emulsion, the dispersing agent and the optional mixed lipid component (F2) is not specifically restricted, and an appropriate mode may be selected. For example, when the F2 is mainly composed of a water-soluble lipid, emulsification can be carried out in a manner that the F2 and the specific dispersing agent are added to W2 in advance, and to the mixture is added the W1/O emulsion. On the other hand, in the case when the F2 is mainly composed of a fat-soluble lipid, emulsification can be carried out in a manner that (after preparation of a W1/O emulsion) the F2 is preliminarily added to the oil phase of the W1/O emulsion, and the mixture is added to W2 to which the specific dispersing agent has been added. In this case, the proportion of the mixed lipid component (F2) added to the aqueous solvent (W2) or the organic solvent (O) of the W1/O emulsion can be properly controlled, while taking use of the finally prepared liposomes, etc, into consideration.

The volume mean particle diameter of the W1/O/W2 emulsion, the proportion of the aqueous solvent (W2), the volume ratio between the W1/O emulsion and the aqueous solvent (W2) and other operation conditions in the secondary emulsification step can be properly controlled, while taking use of the finally prepared liposomes, etc, into consideration.

[Process for Producing Liposomes]

The process for producing liposomes of the present invention further includes the following step (3) in addition to the above-mentioned steps (1) and (2), and, if necessary, other steps, such as steps (4), (5), etc., may be combined therewith, on demand.

That is, the production method of liposomes of the present invention includes a step of forming liposomes by removing the organic solvent (O) contained in the W1/O/W2 emulsion obtained through the steps (1) and (2).

(3) Solvent removal step

The solvent removal step is a step of removing the organic solvent phase (O) contained in the W1/O/W2 emulsion produced in the secondary emulsification step (2) to form liposomes. Examples of methods for removing the solvent include an evaporation method using an evaporator and an in-liquid drying method.

The in-liquid drying method is a method in which the W1/O/W2 emulsion is recovered, transferred into an open container and allowed to stand still or stirred, whereby the organic solvent (O) contained in the W1/O/W2 emulsion is removed by evaporation, and through such operations, a lipid membrane composed of the mixed lipid component (F1) is formed around the inner aqueous phase, and a dispersion of liposomes can be obtained. In this case, removal of the solvent by distillation can be accelerated by heating or pressure reduction. The temperature conditions and the pressure reduction conditions are properly controlled in a conventional manner according to the type of the organic solvent, etc. to be used. The temperature condition is set in a range wherein the solvent does not undergo bumping, and for example, the temperature is preferably in the range of 0 to 60°C, more preferably, 0 to 25°C. The reduced pressure condition is preferably set in the range of a saturated vapor pressure of the solvent to the atmospheric pressure, and more preferably set in the range of a saturated vapor pressure of the solvent + 1% to a saturated vapor pressure of the solvent + 10%. When a mixture of different solvents is used, conditions fitted to a solvent of a higher saturated vapor pressure are preferable. These removal conditions may be combined in a range wherein the solvent does not undergo bumping, and for example, when a drug is used which is heat-labile, the solvent is preferably distilled off under the conditions of a lower temperature and reduced pressure. By stirring the W1/O/W2 emulsion during the solvent removal, the solvent removal proceeds more uniformly. The steps (2) and (3) may be continuously carried out in a manner such that, after the W1/O/W2 emulsion has been prepared by the stirring emulsion method in the step (2), the stirring process is further continued so as to remove the solvent.

In the liposomes produced by the process of the present invention, multivesicular liposomes derived from the W1/O/W2 emulsion may be contained in a certain proportion, and in order to reduce the proportion, it is effective to carry out stirring or pressure reduction, preferably a combination of them. It is important to carry out the pressure reduction and stirring for a period of time longer than that required for removal of most of the solvent. The present inventors speculate that by virtue of this, hydration of the lipid constituting the liposomes proceeds, whereby the multivesicular liposomes are untied and separated into unilamellar liposomes. Moreover, since the multivesicular liposomes which are generated as by-products or remain in the present process have such an inner structure as to contain aqueous droplets having particle diameters derived from the W1/O emulsion, it is possible to convert those liposomes into unilamellar liposomes having the virtually same size as the particle diameter of those particles derived from the W1/O emulsion by allowing them to pass through a filter having a pore diameter slightly greater than the particle size of the W1/O emulsion, in addition to the above-mentioned pressure reducing and stirring processes, or in place of the above-mentioned pressure reducing and stirring processes. More surprisingly, even if these operations are carried out, leakage of the encapsulated substance does not occur. In the case when multivesicular liposomes remain even after such operations, the multivesicular liposomes can be removed by a filter.

The volume mean particle diameter of the liposomes finally produced by the process as described above (granulation step as will be described later may be used, when needed) is not specifically restricted, but when the liposomes are used as liposome formulations for medical treatments, the volume mean particle diameter thereof is preferably in a range of from 50 to 1,000 nm, more preferably, from 50 to 300 nm. The liposomes of such sizes hardly choke blood capillaries and can pass through gaps formed in blood vessels in the vicinity of cancer tissues, so that they are advantageously used when administered to a human body as medicines, etc. For example, by using the W1/O emulsion whose particle diameters have a value in a range from 50 to 300 nm, unilamellar liposomes having the virtually same size as the value can be produced.

### (4) Separation step

The separation step is a step of separating the dispersing agent and liposomes from each other to remove the dispersing agent from the liposome dispersion. For example, if a microfiltration membrane (MF membrane, pore diameter: about 50 nm to 10 $\mu$m) or an ultrafiltration membrane (UF membrane, pore diameter: about 2 to 200 nm) is used, the liposomes can be efficiently separated from the dispersing agent that has formed molecular self-aggregates (volume mean particle diameter of, for example, not more than 10 nm). When there is no problem even if the dispersing agent is not separated from the liposomes in view of use of the product, this separation step does not have to be carried out.

### (5) Other steps

Examples of other steps that are carried out when needed include granulation step and dry-powdering step.

Through the granulation step, the particle diameters of the liposomes prepared can be adjusted to be in the desired range. For example, by the use of a hydrostatic extruder (e.g., "Extruder" manufactured by Nichiyu Liposome Co., Ltd., "Liponizer" manufactured by Nomura Micro Science Co., Ltd.) equipped with a polycarbonate membrane or a cellulose membrane having a pore diameter of 0.1 to 0.4 $\mu$m as a filter, liposomes having a central particle diameter of about 50 to 500 nm are efficiently obtained. If the above "Extruder" or the like is used, multivesicular liposomes secondarily formed from the W1/O/W2 emulsion can be untied into unilamellar liposomes.

It is also desirable that the liposome dispersion is dry-powdered by freeze drying or the like to make its form suitable for storage before use. The freeze drying can be carried out using the same means or device as used in production of conventional liposomes. The freeze drying may be carried out under the appropriate conditions (temperature: -120 to -20°C, pressure: 1 to 15 Pa, time: 16 to 26 hours, etc.) in accordance with, for example, indirect heating freezing method, refrigerant direct expansion method, heating medium circulation method, triple heat exchange method or redundant refrigeration method. By introducing the freeze-dried product obtained as above into water, a dispersion of liposomes can be prepared again.

### EXAMPLES

### (Measuring Method of Particle Size Distribution)

The volume mean particle diameter and CV value of the W1/O emulsion obtained in the primary emulsification step of each of examples and comparative examples were measured by using the following method.

The W1/O emulsion was diluted with a chloroform/hexane mixed solvent (volume ratio: 4/6, the specific gravity was made equal to that of the inner aqueous phase) to 10 times, then a particle size distribution was measured with a dynamic light scattering nanotrack particle size analyzer (UPA-EX150, manufactured by NIKKISO CO. , LTD.), and based on this particle size distribution, a volume mean particle diameter and a CV value (= (standard deviation/volume mean particle diameter) $\times$ 100 [%]) were calculated.

Moreover, the volume mean particle diameter of liposomes was obtained by measuring a produced liposome suspension as it was by the use of the same device.

(Evaluation of Emulsification Behaviors)

Emulsification behaviors of the W1/O/W2 emulsion in the secondary emulsification step were visually observed and evaluated.

○: No continuous outflow of a dispersion phase from a pore passage was observed, with clean droplets being formed, without coalescence of droplets after the formation; thus, a stable operation was carried out.

△: Although a slight continuous outflow of a dispersion phase was observed, clean droplets were formed, without coalescence of droplets after the formation; thus, a stable operation was carried out.

X: A continuous outflow of a dispersion phase from a pore passage was observed, with failure to produce a W1/O/W2 emulsion.

(Evaluation of Encapsulation Rate)

The encapsulation rate of a drug encapsulated in liposomes obtained in examples and comparative examples was measured in accordance with the following method.

(i) Encapsulation rate measuring method for calcein

Total fluorescence intensity ($F_{total}$) of an aqueous solution (3 mL) of liposomes was measured with a spectrophotometer (U-3310, manufactured by JASCO Corporation). Next, 30 $\mu$l of a 0.01 M $CoCl_2$ tris-hydrochloric acid buffer solution was added to quench fluorescence of an encapsulated calcein which had leaked into the outer aqueous phase, by means of $Co^{2+}$, whereby fluorescence intensity ($F_{in}$) inside the liposomes was measured. Moreover, liposomes were produced without addition of calcein under the same conditions as those for the sample, and fluorescence ($F_l$) emitted by the lipid itself was measured. The encapsulation rate was calculated from the following formula.

$$\mathtt{Encapsulation\ rate\ E(\%)\ =\ (F_{in}-F_l)/(F_{total}-F_l)\times 100}$$

(ii) Encapsulation rate measuring method for cytarabine

A solid component (liposomes) was separated (decanted) using an ultracentrifuge, and the amounts of cytarabine contained in the supernatant and the solid component were determined by HPLC for each. The component was assayed by use of a HPLC column (Varian Polaris C18-A (3 $\mu$m, 2$\times$40 mm)). Based upon the absolute values of the corresponding compound that was not encapsulated and the corresponding compound that was encapsulated, the encapsulation rate of cytarabine was calculated.

(Observation Method of Multivesicular Liposomes)

By use of a slide glass with a concave portion, a preparation of a liposome aqueous solution of 25 $\mu$L was formed, and this was observed under a bright viewing field condition of an upright microscope (Axio Imager: A1, made by Carl Zeiss, Ltd.), whereby the number of pieces of the multivesicular liposomes was measured.

Additionally, in the following description, "porous membrane" may be referred to as "emulsification membrane", and "substance to be encapsulated" may be referred to as "encapsulated drug", respectively.

Preparation examples 5, 6, 7, 8, and 9 are not according to the invention.

[Preparation Example 1: Preparation of polysilazane-treated SPG membrane]

A cylindrical SPG membrane having a diameter of 10 mm and a length of 20 mm, with a fine-pore diameter of 10 $\mu$m, commercially available from SPG Technology Co., Ltd., was degreased with an organic solvent (ethanol, etc.),

washed with ultrapure water, and dried, and then subjected to a dipping treatment with a perhydropolysilazane-based treatment solution (Aquamica (registered trademark) NL110A), and after having been dried by heating at 50°C for 5 minutes, this was left for 24 hours or more. Thereafter, by washing this with pure water and then drying by heating at 50°C for 30 minutes, a polysilazane-treated SPG membrane was prepared.

[Preparation Example 2: Preparation of gluconamide-modified SPG membrane]

A cylindrical SPG membrane having a diameter of 10 mm and a length of 20 mm, with a fine-pore diameter of 10 $\mu$m, commercially available from SPG Technology Co., Ltd., was degreased with an organic solvent (ethanol, etc.), washed with ultrapure water, and dried, and then immersed in an N-(3-triethoxysilylpropyl)gluconamide solution (made by Gelest Inc.: catalog number SIT8189.0) diluted with ethanol having a weight of 20 times thereof, and reacted at 50°C for 2 hours, and thereafter, by drying this by heating at 100°C for 3 hours, a gluconamide-modified SPG membrane was prepared.

[Preparation Example 3: Preparation of gluconamide-modified microchannel substrate]

A microchannel, made from silicon (having a terrace length of about 60 $\mu$m, a channel depth of about 11 $\mu$m and a channel width of about 16 $\mu$m), was first subjected to a surface treatment with an oxygen plasma, and further subjected to an acid treatment. The acid treatment of the microchannel was carried out by putting it into a dilute nitric acid to be left therein.
The microchannel, thus preliminarily treated, was then immersed in an N-(3-triethoxysilylpropyl)gluconamide solution (made by Gelest Inc.: catalog number SIT8189.0) diluted by ethanol having a weight of 20 times thereof and reacted at 50°C for 2 hours. Thereafter, by drying this by heating at 100°C for 3 hours, a gluconamide-modified microchannel was prepared.

[Preparation Example 4: Preparation of quaternary ammonium-modified SPG membrane]

A cylindrical SPG membrane having a diameter of 10 mm and a length of 20 mm, with a fine-pore diameter of 10 $\mu$m, commercially available from SPG Technology Co., Ltd., was degreased with an organic solvent (ethanol, etc.), washed with ultrapure water, and dried, and then immersed in an N-trimethoxysilylpropyl-N,N,N-trimethylammonium chloride solution (made by Gelest Inc.: catalog number SIT8415.0; 50% methanol solution) diluted by ethanol having a weight of 10 times thereof, and reacted at 50°C for 2 hours, and thereafter, by drying this by heating at 100°C for one hour, a quaternary ammonium-modified SPG membrane was prepared.

[Preparation Example 5: Preparation of ethylenediamine triacetate-modified SPG membrane]

A cylindrical SPG membrane having a diameter of 10 mm and a length of 20 mm, with a fine-pore diameter of 10 $\mu$m, commercially available from SPG Technology Co., Ltd., was degreased with an organic solvent (ethanol, etc.), washed with ultrapure water, and dried, and then immersed in a trisodium N-(trimethoxysilylpropyl)ethylene diamine triacetate solution (made by Gelest Inc.: catalog number SIT8402.0; 45% aqueous solution) diluted by ethanol having a weight of 10 times thereof, and reacted at 50°C for 2 hours, and thereafter, by drying this by heating at 100°C for 2 hours, an ethylenediamine triacetate-modified SPG membrane was prepared.

[Preparation Example 6: Preparation of diol-modified SPG membrane]

A cylindrical SPG membrane having a diameter of 10 mm and a length of 20 mm, with a fine-pore diameter of 10 $\mu$m, commercially available from SPG Technology Co., Ltd., was degreased with an organic solvent (ethanol, etc.), washed with ultrapure water, and dried, and then immersed in a bis(2-hydroxyethyl)-3-aminopropyl-triethoxysilane solution (made by Gelest Inc.: catalog number SIB1140.0; 62% ethanol solution) diluted by ethanol having a weight of 10 times thereof, and reacted at 50°C for 2 hours, and thereafter, by drying this by heating at 100°C for one hour, a diol-modified SPG membrane was prepared.

[Preparation Example 7: Preparation of disilyl-modified SPG membrane]

A cylindrical SPG membrane having a diameter of 10 mm and a length of 20 mm, with a fine-pore diameter of 10 $\mu$m, commercially available from SPG Technology Co., Ltd., was degreased with an organic solvent (ethanol, etc.), washed with ultrapure water, and dried, and then immersed in a bis(triethoxysilyl)ethane (made by Gelest Inc.: catalog number SIB1817.0) diluted by ethanol having a weight of 20 times thereof, and reacted at 50°C for 2 hours, and thereafter, by drying this by heating at 100°C for one hour, a disilyl-modified SPG membrane was prepared.

[Preparation Example 8: Preparation of octadecyl-modified SPG membrane]

A cylindrical SPG membrane having a diameter of 10 mm and a length of 20 mm, with a fine-pore diameter of 10 $\mu$m, commercially available from SPG Technology Co., Ltd., was degreased with an organic solvent (ethanol, etc.), washed with ultrapure water, and dried, and then immersed in a hexane solution of octadecyltriethoxysilane, and reacted at room temperature for 2 hours, and thereafter, by evaporating hexane through heat-drying processes, an octadecyl-modified SPG membrane was prepared.

[Preparation Example 9: Preparation of octadecyl-modified microchannel substrate]

A microchannel, made from silicon (having a terrace length of about 60 $\mu$m, a channel depth of about 11 $\mu$m and a channel width of about 16 $\mu$m), was first subjected to a surface treatment with an oxygen plasma, and further subjected to an acid treatment. The acid treatment of the microchannel was carried out by putting it into a dilute nitric acid to be left therein.
The microchannel, thus preliminarily treated, was then immersed in a hexane solution of octadecyltriethoxysilane, and reacted at room temperature for 2 hours, and thereafter, by evaporating hexane through heat-drying processes, an octadecyl-modified microchannel substrate was prepared.

[Example 1]

(Production of W1/O emulsion by primary emulsification step)

15 ml of a mixed solvent (hexane/dichloromethane = 7/3) containing 1.5 g of egg yolk lecithin "COATSOME NC-50" (available from NOF CORPORATION) having a phosphatidylcholine content of 95% and 0.25 g of dipalmitoylphosphatidylglycerol (DPPG) was used as an organic solvent phase (O), and 5 ml of a tris-hydrochloric acid buffer solution (pH: 7.4, 50 mmol/L) containing calcein (0.4 mM) was used as an aqueous dispersion phase (W1) for an inner aqueous phase. A mixed liquid of these was placed in a 50 ml beaker and irradiated with ultrasonic waves (output: 5.5) at 25°C for 15 minutes by use of an ultrasonic dispersion device (UH-600S, manufactured by SMT Co., Ltd.) in which a probe having a diameter of 20 mm had been set, to carry out emulsification. As a result of the aforesaid measurement, the W1/O emulsion obtained in this primary emulsification step was confirmed to be a monodisperse W/O emulsion having a volume mean particle diameter of about 190 nm, and the CV value thereof was 33%.

(Production of W1/O/W2 emulsion by secondary emulsification step)

By use of the W1/O emulsion obtained by the above primary emulsification step as a dispersion phase, production of a W1/O/W2 emulsion was carried out by an SPG emulsification method. To an SPG membrane emulsification device (manufactured by SPG Technology Co., Ltd., trade name "External Pressure Type Micro Kit"), a polysilazane-treated SPG membrane prepared in the above-mentioned preparation example 1 was connected, and the outlet side of the device was filled with a tris-hydrochloric acid buffer solution (pH: 7.4, 50 mmol/L) containing a 0.1% Pluronic F-68 serving as an outer aqueous phase solution (W2), while the W1/O emulsion was fed through the inlet side of the device; thus, a W1/O/W2 emulsion was produced.
At this time, no continuous outflow, etc. occurred through the SPG membrane, and clean droplets were consequently formed so that the emulsion was stable even after the emulsification.

(Production of liposomes by removal of organic solvent phase)

Next, the W1/O/W2 emulsion, obtained by the secondary emulsification step, was transferred into a tightly closed container and stirred at room temperature under a reduced pressure of 500 mbar for about 4 hours, and then further stirred at room temperature under a reduced pressure of 180 mbar for about 18 hours so that the solvent was evaporated therefrom. Thus, a suspension of fine liposome particles was obtained, and it was confirmed that calcein was contained in the particles. The average particle diameter of the resultant liposome particles at room temperature was 188 nm. Table 1 shows the calcein encapsulation rate of the liposomes.

[Example 2]

Liposomes were produced in the same manner as in example 1, except that dipalmitoylphosphatidylcholine (DPPC) was used in place of the egg yolk lecithin "COATSOME NC-50". During this process, the emulsification behaviors of the W1/O/W2 emulsion were superior, and no continuous outflow, etc. occurred through the SPG membrane, and clean droplets were formed. The average particle diameter of the resultant liposome particles at room temperature was 202 nm. Table 1 shows the calcein encapsulation rate of the liposomes.

[Example 3]

Liposomes were produced in the same manner as in example 1, except that in the production of a W1/O/W2 emulsion in the secondary emulsification step, a gluconamide-modified SPG membrane prepared in the preparation example 2 was used in place of the polysilazane-treated SPG membrane.

At this time, no continuous outflow, etc. occurred through the SPG membrane, and clean droplets were consequently formed so that the emulsion was stable even after the emulsification. The average particle diameter of the resultant liposome particles at room temperature was 198 nm. Table 1 shows the calcein encapsulation rate of the liposomes.

[Example 4]

Liposomes were produced in the same manner as in example 1, except that dipalmitoylphosphatidylcholine (DPPC) was used in place of the egg yolk lecithin "COATSOME NC-50", and that in the production of a W1/O/W2 emulsion in the secondary emulsification step, a gluconamide-modified SPG membrane prepared in the preparation example 2 was used in place of the polysilazane-treated SPG membrane. During this process, the emulsification behaviors of the W1/O/W2 emulsion were superior, and no continuous outflow, etc. occurred through the SPG membrane, and clean droplets were formed. The average particle diameter of the resultant liposome particles at room temperature was 175 nm. Table 1 shows the calcein encapsulation rate of the liposomes.

[Example 5]

(Production of W1/O emulsion by primary emulsification step)

15 ml of a mixed solvent (hexane/dichloromethane = 7/3) containing 1.5 g of egg yolk lecithin "COATSOME NC-50" (NOF CORPORATION) having a phosphatidylcholine content of 95% and 0.25 g of dipalmitoylphosphatidylglycerol (DPPG) was used as an organic solvent phase (O), and 5 ml of a tris-hydrochloric acid buffer solution (pH: 8, 50 mmol/L) containing calcein (0.4 mM) was used as an aqueous dispersion phase (W1) for an inner aqueous phase. A mixed liquid of these was placed in a 50 ml beaker and irradiated with ultrasonic waves (output: 5.5) at 25°C for 15 minutes by use of an ultrasonic dispersion device (UH-600S, manufactured by SMT Co., Ltd.) in which a probe having a diameter of 20 mm had been set, to carry out emulsification. As a result of the aforesaid measurement, the W1/O emulsion obtained in this primary emulsification step was confirmed to be a monodisperse W/O emulsion having a volume mean particle diameter of about 190 nm, and the CV value thereof was 33%.

(Production of W1/O/W2 emulsion by secondary emulsification step)

Successively, by use of the W1/O emulsion obtained by the above primary emulsification step as a dispersion phase, production of a W1/O/W2 emulsion by a microchannel emulsification method was carried out by the use of a laboratory dead-end type microchannel emulsification device module.

A glass plate was pressure bonded to the gluconamide-modified microchannel substrate prepared in the preparation example 3 to form a channel, and the outlet side of the channel was filled with a tris-hydrochloric acid buffer solution (pH: 7.4, 50 mmol/L) containing a 0.1% Pluronic F-68 serving as an outer aqueous phase solution (W2), while the W1/O emulsion was fed through the inlet side of the device; thus, a W1/O/W2 emulsion was produced. At this time, no continuous outflow, etc. occurred through the SPG membrane, and clean droplets were formed so that the emulsion was stable even after the emulsification.

(Production of liposomes by removal of organic solvent phase)

Next, the W1/O/W2 emulsion, obtained by the secondary emulsification step, was transferred into a tightly closed container and stirred at room temperature under a reduced pressure of 500 mbar for about 4 hours, and then further stirred at room temperature under a reduced pressure of 180 mbar for about 18 hours so that the solvent was evaporated therefrom. Thus, a suspension of fine liposome particles was obtained, and it was confirmed that calcein was contained in the particles. The average particle diameter of the resultant liposome particles at room temperature was 195 nm. Table 1 shows the calcein encapsulation rate of the liposomes.

[Examples 6 to 19]

Liposomes were produced in the same manner as in example 1, except that changes were made as described in Table 1. Emulsification behaviors of the W/O/W emulsion and the encapsulation rates of liposomes thereof are shown in Table 1. In this case, the mixing ratio of hexane and chloroform was set to 7:3 in examples 7 to 10, 13 and 15.

In examples 3, 4, 7, 8, 12, 13 and 16, the gluconamide-modified SPG membrane prepared in the preparation example 2 was used as the emulsification membrane, in examples 5, 6, 9 to 11, 14 and 15, the gluconamide-modified microchannel substrate prepared in the preparation example 3 was used as the emulsification membrane, and in examples 17 to 19, the quaternary ammonium-modified SPG membrane prepared in the preparation example 4 was used as the emulsification membrane, respectively.

[Example 20 (Premix method)]

Liposomes were produced by the same method as that of example 13, except that in the W1/O/W2 emulsion obtained in the secondary emulsification step, a membrane treatment process using an SPG membrane that had not been subjected to a hydrophilicizing treatment was used prior to the organic solvent removing step.

In other words, the SPG membrane that had not been subjected to a hydrophilicizing treatment was used, with the W1/O/W2 emulsion obtained in example 13 serving as a dispersion phase, and the outlet side of the device was filled with a tris-hydrochloric acid buffer solution (pH: 7.4, 50 mmol/L) containing a 3% sodium casein solution serving as the outer aqueous phase solution (W2), with the W1/O/W2 emulsion being fed through the inlet side of the device, so that a membrane treatment was carried out; thus, a final W1/O/W2 emulsion was produced. The removing process of the organic solvent phase was carried out in the same manner as in example 13.

The average particle diameter of the resultant liposomes was 199 nm, and the calcein encapsulation rate was 84%. Moreover, with respect to the W/O/W emulsification behaviors, no continuous outflow, etc. occurred from the pore passage, and clean droplets were formed so that the emulsion was stable without any coalescence of droplets even after the formation.

[Example 21 (Cytarabine)]

(Production of W1/O emulsion by primary emulsification step)

15 ml of a mixed solvent (hexane:dichloromethane = 8:2) containing 0.3 g of egg yolk lecithin "COATSOME NC-50" (NOF CORPORATION) having a phosphatidylcholine content of 95% and 0.152 g of cholesterol (Chol) and 0.108 g of oleic acid (OA) was used as an organic solvent phase (O), and 5 ml of a tris-hydrochloric acid buffer solution (pH: 7.4, 50 mmol/L) containing cytarabine (4 mM) was used as an aqueous dispersion phase (W1) for an inner aqueous phase. A mixed liquid of these was placed in a 50 ml beaker and subjected to the same emulsification step as that of example 1. The average particle diameter of the resultant W1/O emulsion at room temperature was 207 nm.

(Production of W1/O/W2 emulsion by secondary emulsification step)

By use of the W1/O emulsion obtained by the above primary emulsification step as a dispersion phase, production of a W1/O/W2 emulsion was carried out by an SPG emulsification method. In an SPG membrane emulsification device (manufactured by SPG Technology Co., Ltd., trade name "External Pressure Type Micro Kit"), a cylindrical SPG membrane having a diameter of 10 mm, a length of 20 mm, with a fine-pore diameter of 10.0 $\mu$m, was used which had been subjected to a gluconamide-modifying treatment, and the outlet side of the device was filled with a tris-hydrochloric acid buffer solution (pH: 7.4, 50 mmol/L) containing a 3% sodium casein solutionwhich was an outer aqueous phase solution (W2), with the W1/O emulsion being fed through an inlet side of the device, so that a W1/O/W2 emulsion was produced.

(Production of liposomes by removal of organic solvent phase)

Next, the W1/O/W2 emulsion, obtained by the secondary emulsification step, was transferred into a tightly closed container and stirred at room temperature under a reduced pressure of 500 mbar for about 4 hours, and then further stirred at room temperature under a reduced pressure of 180 mbar for about 18 hours so that the solvent was evaporated therefrom. Thus, a suspension of fine liposome particles was obtained, and it was confirmed that cytarabine was contained in the particles. The average particle diameter of the resultant liposome particles at room temperature was 212 nm, and the cytarabine encapsulation rate was 35%, with no multivesicular liposomes being confirmed.

With respect to the W/O/W emulsification behaviors, no continuous outflow of the dispersion phase occurred from the pore passage, and clean droplets were formed so that the emulsion was stable without any coalescence of droplets even after the formation.

In any of the above examples, no multivesicular liposomes were confirmed.

[Comparative Examples 1 to 9]

Liposomes were produced in the same manner as in example 1, except that changes were made as described in Table 1. Emulsification behaviors of the W/O/W emulsion and the encapsulation rates of liposomes thereof are shown in Table 1.

In comparative examples 1 to 3, the ethylenediamine triacetate-modified SPG membrane prepared in the preparation example 5 was used as the emulsification membrane, in comparative example 4, the diol-modified SPG membrane prepared in the preparation example 6 was used as the emulsification membrane, in comparative example 5, the disilyl-modified SPG membrane prepared in the preparation example 7 was used as the emulsification membrane, in comparative examples 6 and 8, the octadecyl-modified SPG membrane prepared in the preparation example 8 was used as the emulsification membrane, and in comparative examples 7 and 9, the octadecyl-modified microchannel substrate prepared in the preparation example 9 was used as the emulsification membrane, respectively.

In the above examples and comparative examples 1 to 9, "polysilazane" described in the column "kind of surface treatment" in Table 1 means that the surface treatment was carried out by using polysilazane, and "gluconamide", "quaternary ammonium", "ethylenediamine triacetate", "diol", "disilyl" and "octadecyl" described therein respectively mean that the surface treatments of the SPG membranes or the microchannels were carried out by use of a silane coupling agent having a gluconamide structure, a silane coupling agent having a quaternary ammonium structure, a silane coupling agent having an ethylene diamine triacetate structure, a silane coupling agent having a diol structure, a silane coupling agent having a disilyl structure, and octadecyltriethoxysilane, as a surface treatment agent, respectively. Moreover, in all of the above examples and comparative examples 7 and 9, in which a microchannel was used as the emulsification membrane, the microchannel was used which had been preliminarily surface-treated by an oxygen plasma and then surface-treated with the above surface treatment agent.

[Comparative Example 10]

(Production of W1/O emulsion by primary emulsification step)

15 ml of a mixed solvent (hexane/dichloromethane = 7/3) containing 1.5 g of egg yolk lecithin "COATSOME NC-50" (NOF CORPORATION) having a phosphatidylcholine content of 95% and 0.25 g of dipalmitoylphosphatidylglycerol (DPPG) was used as an organic solvent phase (O), and 5 ml of a tris-hydrochloric acid buffer solution (pH: 7.4, 50 mmol/L) containing calcein (0.4 mM) was used as an aqueous dispersion phase (W1) for an inner aqueous phase. A mixed liquid of these was placed in a 50 ml beaker and irradiated with ultrasonic waves (output: 5.5) at 25°C for 15 minutes by use of an ultrasonic dispersion device (UH-600S, manufactured by SMT Co., Ltd.) in which a probe having a diameter of 20 mm had been set, to carry out emulsification. As a result of the aforesaid measurement, the W1/O emulsion obtained in this primary emulsification step was confirmed to be a monodisperse W/O emulsion having a volume mean particle diameter of about 190 nm, and the CV value thereof was 33%.

(Production of W1/O/W2 emulsion by secondary emulsification step)

By use of the W1/O emulsion obtained by the above primary emulsification step as a dispersion phase, production of a W1/O/W2 emulsion was carried out by an SPG emulsification method. In an SPG membrane emulsification device (manufactured by SPG Technology Co., Ltd., trade name "External Pressure Type Micro Kit"), a cylindrical SPG membrane having a diameter of 10 mm, a length of 20 mm, with a fine-pore diameter of 10 $\mu$m, was used, and the outlet side of the device was filled with a tris-hydrochloric acid buffer solution (pH: 7.4, 50 mmol/L) containing a 0.1% Pluronic F-68 serving as an outer aqueous phase solution (W2), with the W1/O emulsion being fed through an inlet side of the device, so that an attempt was made to produce a W1/O/W2 emulsion. In this case, the SPG membrane was used without having been particularly subjected to a surface treatment or the like.

At this time, a continuous outflow of the dispersion phase occurred from the SPG membrane absolutely, with failure to successfully form a W1/O/W2 emulsion.

[Comparative Examples 11 to 15, 19 and 20]

Production of liposomes was attempted in the same manner as in comparative example 1 except that each of changes as described in Table 1 was made. In comparative examples 14 and 15, a mixing ratio between hexane and chloroform was set to 7:3. In the productions of the W1/O/W2 emulsion by any of secondary emulsification steps, a continuous outflow of the dispersion phase occurred, with failure to successfully form a W1/O/W2 emulsion.

In this case, in all of the comparative examples 11 to 20, SPG membranes that had not been particularly subjected to a surface treatment or the like were used, and microchannels that had been subjected to only the surface treatment with an oxygen plasma were used, unless otherwise specified.

More specifically, in each of comparative examples 10, 11, 14, 17 and 19, a cylindrical SPG membrane having a diameter of 10 mm and a length of 20 mm, with a fine-pore diameter of 10 $\mu$m, purchased from SPG Technology Co., Ltd. , as it was, was used as its emulsification membrane. On the other hand, in each of comparative examples 12, 13, 15, 16, 18 and 20, a microchannel, made from silicon (having a terrace length of about 60 $\mu$m, a channel depth of about 11 $\mu$m and a channel width of about 16 $\mu$m) was used which had been first surface-treated with an oxygen plasma, and further subjected to an acid treatment, with no other surface treatment being specifically carried out thereon, as its emulsification membrane.

[Comparative Examples 16 to 18]

Production of liposomes was attempted in the same manner as in comparative example 1 except that each of changes as described in Table 1 was made. In any of the cases, although no continuous outflow occurred, emulsification behaviors deteriorated slightly, and the encapsulation rates were lowered in comparison with the encapsulation rates of examples 11, 12 and 14 of the present invention.

[Comparative Examples 21]

Liposomes were produced by the same method as that of example 20 except that in the SPG membrane emulsification device, an SPG membrane that had not been subjected to a gluconamide treatment was used as a cylindrical SPG membrane in the secondary emulsification step. The average particle diameter of the resultant liposome particles was 220 nm, and the calcein encapsulation rate was 66%, with no multivesicular liposomes being found.

With respect to the W/O/W emulsification behaviors, clean droplets were formed in a stable manner, without any coalescence of droplets after the formation; however, a continuous outflow of the dispersion phase was slightly observed.

[Comparative Example 22]

Liposomes were produced by the same method as that of example 21 except that in the SPG membrane emulsification device, an SPG membrane that had not been subjected to a gluconamide treatment was used as a cylindrical SPG membrane in the secondary emulsification step. The average particle diameter of the resultant liposome particles was 202 nm, and the cytarabine encapsulation rate was 27%, with no multivesicular liposomes being found.

With respect to the W/O/W emulsification behaviors, clean droplets were formed in a stable manner, without any coalescence of droplets after the formation; however, a continuous outflow of the dispersion phase was slightly observed.

The results of the above-mentioned examples and comparative examples are shown in the following Table 1.

[Table 1-1] <Table 1 (no. 1)>

| | Emulsification membrane | Kinds of surface treatment | Contact angle (°) (Immediately after surface treatment) | Encapsulated drug | Lipid composition | Oil phase solvent | Dispersing agent in outer aqueous phase | Membrane treatment of W/O/W emulsion | Emulsification behaviors of W/O/W emulsion | Encapsulation rate of drug in liposomes (%) |
|---|---|---|---|---|---|---|---|---|---|---|
| Example 1 | SPG | Polysilazane | 17 | calcein | Egg yolk lecithin/ DPPG | hexane/ dichloromethane | Pluronic F-68 | No | ○ | 80 |
| Example 2 | SPG | Polysilazane | 17 | calcein | DPPC/DPPG | hexane/ dichloromethane | Pluronic F-68 | No | ○ | 85 |
| Example 3 | SPG | gluconamide | 35 | calcein | Egg yolk lecithin/ DPPG | hexane/ dichloromethane | Pluronic F-68 | No | ○ | 85 |
| Example 4 | SPG | gluconamide | 35 | calcein | DPPC/DPPG | hexane/ dichloromethane | Pluronic F-68 | No | ○ | 83 |
| Example 5 | microchannel | gluconamide | 35 | calcein | Egg yolk lecithin/ DPPG | hexane/ dichloromethane | Pluronic F-68 | No | ○ | 84 |
| Example 6 | microchannel | gluconamide | 35 | calcein | DPPC/DPPG | hexane/ dichloromethane | Pluronic F-68 | No | ○ | 82 |
| Example 7 | SPG | gluconamide | 35 | calcein | Egg yolk lecithin/ DPPG | hexane/ chloroform | Pluronic F-68 | No | ○ | 82 |
| Example 8 | SPG | gluconamide | 35 | calcein | DPPC/DPPG | hexane/ chloroform | Pluronic F-68 | No | ○ | 82 |
| Example 9 | microchannel | gluconamide | 35 | calcein | Egg yolk lecithin/ DPPG | hexane/ chloroform | Pluronic F-68 | No | ○ | 83 |
| Example 10 | microchannel | gluconamide | 35 | calcein | DPPC/DPPG | hexane/ chloroform | Pluronic F-68 | No | ○ | 83 |
| Example 11 | microchannel | gluconamide | 35 | calcein | Egg yolk lecithin/ DPPG | hexane | Pluronic F-68 | No | ○ | 88 |

| | Emulsification membrane | Kinds of surface treatment | Contact angle (°) (Immediately after surface treatment) | Encapsulated drug | Lipid composition | Oil phase solvent | Dispersing agent in outer aqueous phase | Membrane treatment of W/O/W emulsion | Emulsification behaviors of W/O/W emulsion | Encapsulation rate of drug in liposomes (%) |
|---|---|---|---|---|---|---|---|---|---|---|
| Example 12 | SPG | gluconamide | 35 | calcein | Egg yolk lecithin/ DPPG | hexane/ dichloromethane | casein Na | No | ○ | 88 |
| Example 13 | SPG | gluconamide | 35 | calcein | Egg yolk lecithin/ DPPG | hexane/ chloroform | casein Na | No | ○ | 87 |
| Example 14 | microchannel | gluconamide | 35 | calcein | Egg yolk lecithin/ DPPG | hexane/ dichloromethane | casein Na | No | ○ | 89 |
| Example 15 | microchannel | gluconamide | 35 | calcein | Egg yolk lecithin/ DPPG | hexane/ chloroform | casein Na | No | ○ | 87 |
| Example 16 | SPG | gluconamide | 35 | calcein | Egg yolk lecithin/ DPPG | hexane/ dichloromethane | purified geratin | No | ○ | 80 |

[Table 1-2] <Table 1 (no. 2)>

| | Emulsification membrane | Kinds of surface treatment | Contact angle (°) (Immediately after surface treatment) | Encapsulated drug | Lipid composition | Oil phase solvent | Dispersing agent in outer aqueous phase | Membrane treatment of W/O/W emulsion | Emulsification behaviors of W/O/W emulsion | Encapsulation rate of drug in liposomes (%) |
|---|---|---|---|---|---|---|---|---|---|---|
| Example 17 | SPG | quaternary ammonium | 42 | calcein | Egg yolk lecithin/DPPG | hexane/ dichloromethane | casein Na | No | ○ | 85 |
| Example 18 | SPG | quaternary ammonium | 42 | calcein | Egg yolk lecithin/DPPG | hexane/ dichloromethane | purified gelatin | No | ○ | 83 |
| Example 19 | SPG | quaternary ammonium | 42 | calcein | Egg yolk lecithin/DPPG | hexane/ dichloromethane | Pluronic F-68 | No | ○ | 85 |
| Example 20 | SPG | gluconamide | 35 | calcein | Egg yolk lecithin/DPPG | hexane/ chloroform | casein Na | Yes | ○ | 84 |
| Example 21 | SPG | gluconamide | 35 | cytarabine | Egg yolk lecithin/ Chol/ Oleic acid | hexane/ dichloromethane | casein Na | No | ○ | 35 |
| Comparative example 1 | SPG | ethylenediamine triacetate | 43 | calcein | Egg yolk lecithin/DPPG | hexane/ dichloromethane | Pluronic F-68 | No | Δ | 49 |
| Comparative example 2 | SPG | ethylenediamine triacetate | 43 | calcein | Egg yolk lecithin/DPPG | hexane/ dichloromethane | purified gelatin | No | X | 44 |
| Comparative example 3 | SPG | ethylenediamine triacetate | 43 | calcein | Egg yolk lecithin/DPPG | hexane/ dichloromethane | casein Na | No | Δ | 70 |
| Comparative example 4 | SPG | diol | 51 | calcein | Egg yolk lecithin/DPPG | hexane/ dichloromethane | Pluronic F-68 | No | Δ | 41 |
| Comparative example 5 | SPG | disilyl | 80 | calcein | Egg yolk lecithin/DPPG | hexane/ dichloromethane | Pluronic F-68 | No | X | - |
| Comparative example 6 | SPG | octadecyl | 126 | calcein | Egg yolk lecithin/DPPG | hexane/ dichloromethane | Pluronic F-68 | No | X | - |
| Comparative example 7 | microchannel | octadecyl | 126 | calcein | Egg yolk lecithin/DPPG | hexane/ dichloromethane | Pluronic F-68 | No | X | - |
| Comparative example 8 | SPG | octadecyl | 126 | calcein | Egg yolk lecithin/DPPG | hexane/ dichloromethane | casein Na | No | X | - |

| | Emulsification membrane | Kinds of surface treatment | Contact angle (°) (Immediately after surface treatment) | Encapsulated drug | Lipid composition | Oil phase solvent | Dispersing agent in outer aqueous phase | Membrane treatment of W/O/W emulsion | Emulsification behaviors of W/O/W emulsion | Encapsulation rate of drug in liposomes (%) |
|---|---|---|---|---|---|---|---|---|---|---|
| Comparative example 9 | microchannel | octadecyl | 126 | calcein | Egg yolk lecithin/DPPG | hexane/ dichloromethane | casein Na | No | X | - |

[Table 1-3] <Table 1 (no. 3)>

| | Emulsification membrane | Kinds of surface treatment | Contact angle (°) (Immediately after surface treatment) | Encapsulated drug | Lipid composition | Oil phase solvent | Dispersing agent in outer aqueous phase | Membrane treatment of W/O/W emulsion | Emulsification behaviors of W/O/W emulsion | Encapsulation rate of drug in liposomes (%) |
|---|---|---|---|---|---|---|---|---|---|---|
| Comparative example 10 | SPG | Untreated | | calcein | Egg yolk lecithin/ DPPG | hexane/ dichloromethane | Pluronic F-68 | No | X | - |
| Comparative example 11 | SPG | Untreated | | calcein | DPPC/DPPG | hexane/ dichloromethane | Pluronic F-68 | No | X | - |
| Comparative example 12 | microchannel | Untreated | | calcein | Egg yolk lecithin/ DPPG | hexane/ dichloromethane | Pluronic F-68 | No | X | - |
| Comparative example 13 | microchannel | Untreated | | calcein | DPPC/DPPG | hexane/ dichloromethane | Pluronic F-68 | No | X | - |
| Comparative example 14 | SPG | Untreated | | calcein | Egg yolk lecithin/ DPPG | hexane/ chloroform | Pluronic F-68 | No | X | - |
| Comparative example 15 | microchannel | Untreated | | calcein | Egg yolk lecithin/ DPPG | hexane/ chloroform | Pluronic F-68 | No | X | - |
| Comparative example 16 | microchannel | Untreated | | calcein | Egg yolk lecithin/ DPPG | hexane | Pluronic F-68 | No | Δ | 59 |
| Comparative example 17 | SPG | Untreated | | calcein | Egg yolk lecithin/ DPPG | hexane/ dichloromethane | casein Na | No | Δ | 80 |
| Comparative example 18 | microchannel | Untreated | | calcein | Egg yolk lecithin/ DPPG | hexane/ dichloromethane | casein Na | No | Δ | 81 |
| Comparative example 19 | SPG | Untreated | | calcein | Egg yolk lecithin/ DPPG | hexane/ dichloromethane | purified geratin | No | X | - |

| | Emulsification membrane | Kinds of surface treatment | Contact angle (°) (Immediately after surface treatment) | Encapsulated drug | Lipid composition | Oil phase solvent | Dispersing agent in outer aqueous phase | Membrane treatment of W/O/W emulsion | Emulsification behaviors of W/O/W emulsion | Encapsulation rate of drug in liposomes (%) |
|---|---|---|---|---|---|---|---|---|---|---|
| Comparative example 20 | microchannel | Untreated | | calcein | Egg yolk lecithin/ DPPG | hexane/ dichloromethane | purified geratin | No | X | - |
| Comparative example 21 | SPG | Untreated | | calcein | Egg yolk lecithin/ DPPG | hexane/ chloroform | casein Na | Yes | Δ | 66 |
| Comparative example 22 | SPG | Untreated | | cytarabine | Egg yolk lecithin/ Chol/ Oleic acid | hexane/ dichloromethane | casein Na | No | Δ | 27 |

EP 2 502 617 B1

As described above, in examples in which the surface treatment was carried out by use of polysilazane (examples 1 and 2), as well as in examples in which the surface treatment was carried out by use of a silane coupling agent having a hydroxyl group or a cationic group as a hydrophilic group (examples 3 to 19), it was confirmed that by setting a contact angle of a penetration pore of the membrane emulsification device with water in a range from 0° or more to 42° or less, no continuous outflow occurred upon production of a W1/O/W2 emulsion by a secondary emulsification step, and clean droplets were formed, with superior emulsification behaviors, and that it consequently became possible to maintain a high encapsulation rate of 80% or more with respect to the encapsulation rate of liposomes. In contrast, in the case when a membrane emulsification device which did not make the contact angle of a penetration pore of the membrane emulsification device with water in a range from 0° or more to 42° or less even after surface treatment with a surface treatment agent was used, as well as in the case when a membrane emulsification device that had not been surface-treated with a surface treatment agent was used, it was not possible to form a W1/O/W2 emulsion itself in almost all the comparative examples and liposomes could not be obtained. Even in the comparative examples where liposomes could be formed, when a Pluronic F-68 was used as the dispersing agent, the encapsulation rate was lowered (comparative example 16). In the case when sodium (Na) casein was used as the dispersing agent (comparative examples 17 and 18), although the formation of liposomes, itself, was desirably carried out, it was confirmed that tendency of the occurrence of a continuous outflow was observed in comparison with the corresponding examples in which a membrane emulsification device subjected to a surface treatment with a surface treatment agent was used (examples 12 and 14). It is considered that since sodium (Na) casein itself has not been approved as an injection drug, the fact that a high encapsulation rate of 80% or more of liposomes is achieved without using the sodium (Na) casein devotes greatly to achieving the liposome DDS, and that great effects can be expected. Moreover, another remarkable advantage is that the corresponding effects can be obtained irrespective of the kinds of a solvent. Furthermore, although the contact angle (43°) obtained in the case of the surface treatment with ethylenediamine triacetate is virtually the same as the contact angle (42°) obtained in the case of the surface treatment with quaternary ammonium, the former tends to cause a continuous outflow, with failure to provide expected liposomes (comparative examples 1 to 3). It is speculated that the reason for this is because the effect of a carboxyl group exceeds that of an amine group, with the result that the same expected effect as that of the quaternary ammonium is not sufficiently exerted.

When the process in which the premix method was applied, that is, the process in which the resultant W/O/W emulsion was further membrane-treated by the secondary emulsification step, was examined, a high encapsulation rate was maintained even after the prepared W/O/W emulsion had been further subjected to a membrane-treatment, in the case when a porous membrane subjected to a surface treatment with a surface treatment agent was used (example 20). The value of this encapsulation rate was comparable to that obtained in the case when no membrane treatment was carried out on the corresponding W/O/W emulsion (example 13). In contrast, in the case when a porous membrane that had not been subjected to a surface treatment with the surface treatment agent was used, the encapsulation rate was lowered, and the resulting value became even lower than that obtained in the case when no membrane treatment was carried out on the corresponding W/O/W emulsion (example 17).

When the process in which the drug to form a substance to be encapsulated was changed from calcein to cytarabine was examined, a superior encapsulation rate was obtained in the case of using porous membrane subjected to a surface treatment with a surface treatment agent (example 21) in comparison with the process in which a porous membrane that had not been subjected to a surface treatment with a surface treatment agent was used (comparative example 22).

Additionally, in both of comparative examples 21 and 22, tendency of a continuous outflow was observed.

Moreover, although not shown in examples, in the case when sunflower oil was used as a solvent forming the organic phase (O), the removal of this organic solvent was difficult and liposomes were not obtained.

## Claims

1. A process for producing liposomes comprising the following steps (1) to (3):

    (1) a primary emulsification step of: emulsifying an organic solvent (O) that is a volatile organic solvent, an aqueous solvent (W1) and a mixed lipid component (F1), to give a W1/O emulsion;
    (2) a secondary emulsification step of: dispersing the W1/O emulsion produced in the step (1) in an aqueous solvent (W2) by use of a porous membrane, to give the W1/O/W2 emulsion; and
    (3) a solvent removal step of: removing the organic solvent (O) contained in the W1/O/W2 emulsion, to form a unilamellar liposome,

    wherein the porous membrane has been surface-treated with a hydrophilic drug so as to allow its surface to have a contact angle with water in the air in a range from 0° or more to 42° or less,

wherein the hydrophilic drug is a silica precursor monomer or a silane coupling agent having a hydrophilic group on the surface thereof, the silane coupling agent containing at least one structure selected from the group consisting of a saccharide analog structure, an alicyclic polyol structure, a polyether structure, a polyamine structure and a quaternary ammonium structure, as the hydrophilic group,

wherein the mixed lipid component (F1) is at least one selected from the group consisting of a phospholipid, a sterol, a glycolipid, a glycol, an aliphatic amine and a longchain aliphatic acid,

wherein in the step (1), the organic solvent (O) is an organic solvent having a boiling point of less than 100°C and the emulsification is carried out with a substance to be encapsulated further contained in the aqueous solvent (W1),

wherein the step (2) is carried out with a dispersing agent further contained in the aqueous solvent (W2), and

wherein the dispersing agent has a weight-average molecular weight of in a range of from 1,000 to 100,000.

2. The process according to Claim 1, wherein the silane coupling agent contains at least one structure selected from the group consisting of a saccharide analog structure, an alicyclic polyol structure and a quaternary ammonium structure, as the hydrophilic group.

3. The process according to Claim 1, wherein the dispersing agent is at least one material selected from the group consisting of protein, polysaccharides and nonionic surface active agents.

4. The process according to any one of Claims 1 to 3, wherein the porous membrane is in the form of an SPG membrane or a microchannel substrate.

5. The process according to Claim 1, wherein in the step (1), the emulsification is carried out with a substance to be encapsulated further contained in the aqueous solvent (W1), and wherein a drug for medical treatments is used as the substance to be encapsulated, and the resultant liposomes have a volume mean particle diameter of from 50 to 300 nm.

6. The process for producing liposomes according to Claim 1, further comprising a step of separating the dispersing agent and liposomes from each other.

**Patentansprüche**

1. Verfahren zur Herstellung von Liposomen mit den folgenden Schritten (1) bis (3):

(1) ein primärer Emulgierschritt: Emulgieren eines organischen Lösemittels (O), das ein flüchtiges organisches Lösemittel ist, eines wässrigen Lösemittels (W1) und einer gemischten Lipidkomponente (F1) zur Herstellung einer W1/O-Fmulsion:

(2) ein sekundärer Emulgierschritt: Dispergieren der in Schritt (1) erzeugten W1/O-Fmulsion in einem wässrigen Lösemittel (W2) unter Verwendung einer porösen Membran zur Herstellung der W1/O/W2-Emuision; und

(3) ein Lösemittel-Entfernschritt: Entfernen des in der W1/O/W2-Emuision enthaltenen organischen Lösemittels (O) zur Ausbildung eines unilamellaren Liposoms,

wobei die poröse Membran mit einem hydrophilen Mittel oberflächenbehandelt wurde, um zu ermöglichen, dass ihre Oberfläche einen Kontaktwinkel mit Wasser ungefähr in einem Bereich von 0° oder mehr bis 42° oder weniger aufweist,

wobei das hydrophile Mittel aus einem Monomer eines Kieselsäurevorprodukts oder einem Silanhaftvermittler besteht, der eine hydrophile Gruppe an dessen Oberfläche aufweist, wobei der Silanhaftvermittler zumindest eine Struktur als hydrophile Gruppe enthält, die ausgewählt ist aus der Gruppe, die aus einer Sacharid-Analogstruktur, einer alizyklischen Polyalkoholstruktur, einer Polyetherstruktur, einer Polyaminstruktur und einer quatären Ammoniumstruktur besteht,

wobei die gemischte Lipidkomponente (F1) zumindest aus einem Element besteht, das aus der Gruppe ausgewählt wurde, die aus einem Phospholipid, einem Sterin, einem Glykolipid, einem Glykol, einem aliphatischen Amin und einer langkettigen aliphatischen Säure besteht,

wobei in Schritt (1) das organische Lösemittel (O) aus einem organischen Lösemittel mit einem Siedepunkt von weniger als 100°C besteht und die Emulgierung mit einer ferner in dem wässrigen Lösemittel (W1) enthaltenen einzukapselnden Substanz durchgeführt wird,

wobei der Schritt (2) mit einem ferner in dem wässrigen Lösemittel (W2) enthaltenen Dispersionsmittel durchgeführt wird, und

wobei das Dispersionsmittel ein durchschnittliches Molekulargewicht in einem Bereich von 1.000 bis 100.000 aufweist.

**2.** Verfahren gemäß Anspruch 1, wobei der Silanhaftvermittler zumindest eine Struktur als hydrophile Gruppe aufweist, die ausgewählt ist aus der Gruppe, die aus einer Sacharid-Analaogstruktur, einer alizyklischen Polyalkoholstruktur und einer quartären Ammoniumstruktur besteht.

**3.** Verfahren gemäß Anspruch 1, wobei das Dispersionsmittel zumindest aus einem Material besteht, das ausgewählt ist aus der Gruppe, die aus Protein, Polysacchariden und nichtionischen Tensiden besteht.

**4.** Verfahren gemäß irgendeinem der Ansprüche 1 bis 3, wobei die poröse Membran in der Form einer SPG-Membran oder eines Mikrokanal-Substrats ausgebildet ist.

**5.** Verfahren gemäß Anspruch 1, wobei in Schritt (1) die Emulgierung mit einer ferner in dem wässrigen Lösemittel (W1) enthaltenen einzukapselnden Substanz durchgeführt wird, und wobei ein Arzneimittel für medizinische Behandlungen als einzukapselnde Substanz verwendet wird und die resultierenden Liposome einen mittleren Volumenpartikeldurchmesser von 50 bis 300 nm aufweisen.

**6.** Verfahren zur Herstellung von Liposomen gemäß Anspruch 1, ferner aufweisend einen Schritt zur Trennung des Dispersionsmittels und der Liposome voneinander.


**Revendications**

**1.** Procédé de fabrication de liposomes comprenant les étapes suivantes (1) à (3) :

(1) une étape d'émulsification primaire de: émulsifier un solvant organique (O), qui est un solvant organique volatile, un solvant aqueux (W1) et un composant lipidique mélangé (F1) pour produire une émulsion W1/0 ;
(2) une étape d'émulsification secondaire de: disperser l'émulsion W1/O produite dans l'étape (1) dans un solvant aqueux (W2) en utilisant une membrane poreuse pour produire l'émulsion W1/O/W2 ; et
(3) une étape d'élimination de solvant de : éliminer le solvant organique (O) contenu dans l'émulsion W1/O/W2 pour former un liposome unilamellaire,

dans lequel la membrane poreuse a été traitée en surface avec un agent hydrophile pour permettre à sa surface d'avoir un angle de contact avec de l'eau compris dans une plage d'environ 0° ou plus à 42° ou moins,
dans lequel l'agent hydrophile est un monomère de précurseur de silice ou un agent de couplage en silane ayant un groupe hydrophile sur la surface de celui-ci, l'agent de couplage en silane contenant au moins une structure sélectionnée dans le groupe composé d'une structure analogue de saccharide, d'une structure de polyol alicyclique, d'une structure de polyéther, d'une structure de polyamine et d'une structure d'ammonium quaternaire comme groupe hydrophile,
dans lequel le composant lipidique mélangé (F1) est au moins un élément sélectionné dans le groupe composé d'un phospholipide, d'un stérol, d'un glycolipide, d'un glycol, d'un amine aliphatique et d'un acide aliphatique à longue chaîne,
dans lequel dans l'étape (1) le solvant organique (O) est un solvant organique ayant un point d'ébullition inférieur à 100°C et l'émulsification est effectuée avec une substance à encapsuler, qui est en outre contenue dans le solvant aqueux (W1),
dans lequel l'étape (2) est effectuée avec un agent de dispersion, qui est en outre contenu dans le solvant aqueux (W2), et
dans lequel l'agent de dispersion a un poids moléculaire moyen compris dans une gamme de 1.000 à 100.000.

**2.** Procédé selon la revendication 1, dans lequel l'agent de couplage en silane contient au moins une structure sélectionnée dans le groupe composé d'une structure analogue de saccharide, d'une structure de polyol alicyclique et d'une structure d'ammonium quaternaire comme groupe hydrophile.

**3.** Procédé selon la revendication 1, dans lequel l'agent de dispersion est au moins un matériau sélectionné dans le groupe composé de protéine, de polysaccharides et d'agents tensio-actifs anioniques.

**4.** Procédé selon l'une quelconque des revendications 1 à 3, dans lequel la membrane poreuse est configurée en forme d'une membrane SPG ou en forme d'un substrat à micro-canaux.

5. Procédé selon la revendication 1, dans lequel dans l'étape (1) l'émulsification est effectuée avec une substance à encapsuler, qui est en outre contenue dans le solvant aqueux (W1) et dans lequel un agent destiné aux traitements médicaux est utilisé comme substance à encapsuler, et les liposomes résultant comprennent un diamètre de particule de volume moyen compris entre 50 et 300 nm.

6. Procédé de fabrication de liposomes selon la revendication 1, comprenant en outre une étape de séparer l'agent de dispersion des liposomes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5326484 A
- US 2007248541 A
- JP 4009733 B
- JP 2001505549 A
- JP 2001522870 A
- JP 2003071261 A
- JP S5246382 A

**Non-patent literature cited in the description**

- *J Dispers Sci Technol,* 1988, vol. 9 (1), 1-15
- Influences of Aqueous Phase Composition on Lipid Capsule Formation Using Multiphase Emulsion as Base. **TAKASHI KUROIWA ; MITSUTOSHI NAKAJIMA ; SOSAKU ICHIKAWA.** Summary of the 74th Annual Meeting of the Society of Chemical Engineers. March 2009